# EUROPEAN PATENT APPLICATION

(11) **EP 3 243 816 A1**
(43) Date of publication of application: **15.11.2017**
(21) Application number: 16169055.7
(22) Date of filing: 10.05.2016
(51) Int. Cl.: C07D 241/02

(54) **PROCESS FOR PREPARING A SUBSTITUTED AROMATIC OR HETEROAROMATIC HYDROCARBON AND ITS USE**

(71) Applicant: Studiengesellschaft Kohle mbH, 45470 Mülheim an der Ruhr (DE); President and Fellows of Harvard College, Cambridge, MA 02138 (US)
(72) Inventor: RITTER, Tobias, 45470 Muelheim (DE); SERPIER, Fabien, 87240 Ambazac (FR); BOURSALIAN, Greg, Newbury Park, CA California 91320 (US)
(74) Representative: Nobbe, Matthias

(57) **Abstract**

The present invention refers to a process making use of a radical aromatic substitution reaction for preparing an Ar-N-R¹-*N*-diazoniabicyclo[2.2.2]octane dianion salt, wherein an aromatic hydrocarbon Ar is bonded to a 1-R-1,4-diazabicyclo[2.2.2]octane²⁺] 2X⁻ compound, X being an anion.

## Description

The present invention refers to a process making use of a radical aromatic substitution reaction for preparing an Ar-*N*-R¹-*N*'-diazoniabicyclo[2.2.2]octane salt, wherein an aromatic or heteroaromatic hydrocarbon Ar is bonded to a 1-R¹-1,4-diazabicyclo[2.2.2]octane²⁺] 2X⁻ compound, X being an anion. The obtained compound can be further reacted to a 4-substituted piperazine of the formula 4-Ar-piperazine.

Historically, electrophilic aromatic substitution is perhaps the most important reaction class for the functionalization of aromatic C-H bonds, but typically affords mixtures of products. Transition-metal catalyzed reactions have generally struggled with the same limitations in positional selectivity, except when a coordinating directing group on the arene substrate is utilized to position the catalyst within close proximity to a specific C-H bond.

Steric hindrance has been explored as a strategy to control positional selectivity in C-H functionalization, but product mixtures still result, particularly for monosubstituted arenes. There have been isolated reports of non-chelation-assisted aromatic C-H functionalization reactions with anomalously high *para* selectivity for monosubstituted arenes; however, these reactions either require solvent quantities of arene or work only on activated arenes, and the origin of their para selectivity is unknown, precluding generalization to the design of other *para* selective functionalization reactions.

Thus, no general strategy currently exists for highly *para* selective C-H functionalization. Such a strategy would constitute a novel complement to the classical electrophilic aromatic substitution paradigm, especially if no particular directing group is required.

The inventors now found that aromatic substitution by highly electrophilic radicals, which are capable of eliciting significant charge transfer from the arene in the transition state of addition, exhibits high reactivity and a high selectivity for positions *para* to substituents on the arene. Radical aromatic substitution reactions normally do not proceed with synthetically useful positional selectivity on substituted arenes. The results reported herein demonstrate that, contrary to prior assumptions, radical aromatic substitution can furnish novel, useful products with high chemo- and positional selectivity when an appropriately electrophilic radical is used. The inventors have shown that for most substrates, including monosubstituted arenes, only one of the possible positional isomers is observed in significant amounts. The charge transfer directed concept does not require a coordinating directing group as do chelation-assisted C-H functionalization reactions because selectivity is determined by the electronic structure in the transition state as opposed to enforced proximity of the catalyst.

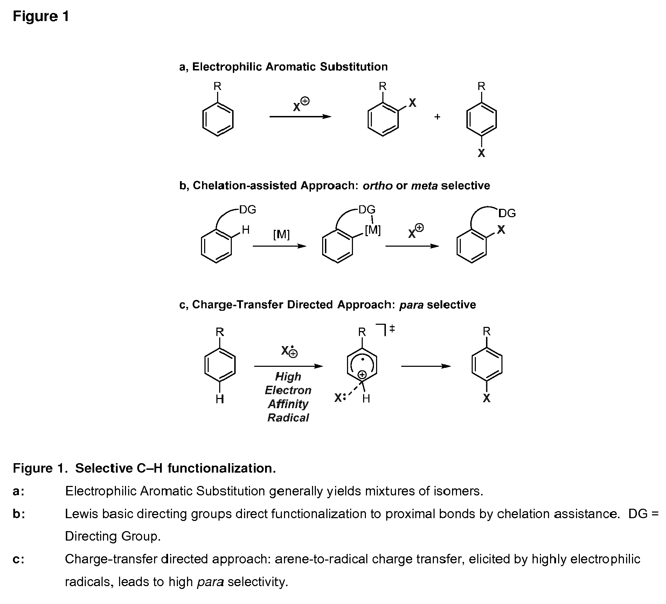

Thus, the reaction developed by the inventors allows the functionalization of aromatic rings with a TEDA moiety, with an excellent and predictable selectivity, predicted by Fukui nucleophilicity indices. For simple aromatic rings possessing one substituent, the substitution is para-selective. Starting from these precursors, several TEDA derivatives have been synthetized with good to high yields, illustrating this new reactivity in Figure 1c. As an application of this reaction, the grafted TEDA moiety can be then converted into a piperazine, an important pharmacophore, after addition of a saturated aqueous solution of Na₂S₂O₃ and subsequent heating.

As it is illustrated in Figure 1:
a: electrophilic aromatic substitution generally yields mixtures of isomers
b: Lewis basic directing groups direct functionalization to proximal bonds by chelation assistance. DG = Directing Group, and
c: Charge-transfer directed approach of the present invention: arene-to-radical charge transfer, elicited by highly electrophilic radicals, leads to high *para* selectivity

Thus, the present invention is directed to an Ar-N-R¹-N'-diazoniabicyclo[2.2.2]octane salt of formula (I), 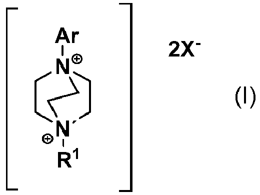 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or C₅ to C₂₀ heteroaryl-C₆ to C₂₀-alkyl group, each group R¹ being optionally substituted, and
X- is an anion.

In specific embodiments of the inventive compound of Formula (I), wherein Ar and X have the meanings as given above, the substituent R¹ may not have any hydrogen beta to the N'- nitrogen as an elimination reaction might take place. The skilled man will be in the position to find substituents and/or reaction conditions in which such generally undesired elimination will be prevented. In any case, the present invention is also covering said Ar-*N*-R¹-*N*'-diazoniabicyclo[2.2.2]octane salt of formula (I) with the proviso that R1 does not have any hydrogen beta to the *N*'-nitrogen, wherein Ar, R¹ and X have the meanings as given above.

In one embodiment of the invention, the Ar-*N* R'-*N*-diazoniabicyclo[2.2.2]octane salt of formula (I) is comprising a mono- or polycyclic aromatic or heteroaromatic hydrocarbon Ar which is substituted by one or more substituents R² which may be, independently from each other, the same or different and is each selected from hydrogen (also deuterium), halogen, straight chain, branched chain or cyclic aliphatic C₁ to C₂₀ alkyl group, optionally having one or more unsaturated bonds, C₆ to C₂₀ aryl or C₆ to C₂₀ aralkyl group or C₅ to C₂₀ heteroaryl or C₅ to C₂₀ heteroaryl-C₆ to C₂₀-alkyl group, R² optionally bonded via -O-and/or R² optionally being substituted by one or more groups selected from halogen, straight chain, branched chain or cyclic aliphatic C₁ to C₂₀ alkyl group, optionally having one or more unsaturated bonds, C₆ to C₂₀ aromatic hydrocarbons or C₅ to C₂₀ heteroaromatic hydrocarbons, C₆ to C₂₀ aryl-(C₁-C₆)-alkyl, or C₅ to C₂₀ heteroaryl-(C₁-C₆)-alkyl, or at least two R² are forming a ring system selected from cyclic aliphatic C₄ to C₂₀ hydrocarbons, C₆ to C₂₀ aromatic hydrocarbons or C₅ to C₂₀ heteroaromatic hydrocarbons, each being optionally substituted.,

The mono- or polycyclic aromatic or heteroaromatic hydrocarbon Ar may be bonded either directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof. In the simplest form, the polymer is comprising a polymeric chain, including heteroatoms in the chain, to which at least one mono- or polycyclic aromatic or heteroaromatic hydrocarbon is bound. A linking group may have the meaning of R² except hydrogen or halogen.

Such a polymer may comprise more than one Ar-N-R¹-N'-diazoniabicyclo[2.2.2]octane salt unit, which are bonded either directly or via a linking group to the polymer. A linking group may have the meaning of R² except hydrogen or halogen.

Thus, the present invention also covers such polymer, preferably selected from polyalkylenes such as polyethylene, polypropylene, polybutadiene, substituted polyalkylenes or copolymers thereof, comprising at least two Ar-N-R¹-N'-diazoniabicyclo[2.2.2]octane salt units of formula (I), 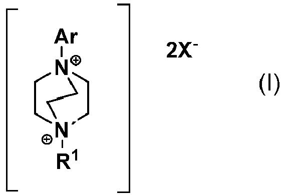 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or heteroaryl-C₆ to C₂₀-alkyl group, each group being optionally substituted, and
X- is an anion.

In one embodiment of the present invention, the Ar-N-R¹-N'-diazoniabicyclo[2.2.2]octane salt of formula (I), R¹ may be a straight chain, branched chain or cyclic aliphatic C₁ to C₆ alkyl group, a C₆ to C₁₀ aryl or aralkyl group or C₅ to C₉ heteroaryl or heteroarylalkyl group, each group being optionally substituted.

In the Ar-N-R¹-N'-diazoniabicyclo[2.2.2]octane salt of formula (I), the anion is not very decisive as long as the reaction is not negatively influenced. Preferably, X- may be an anion selected from halogen, triflate, BF₄⁻, SbF₆⁻, PtF₆⁻.

The invention is also directed to a process for preparing an Ar-N-R¹-N'-diazoniabicyclo[2.2.2]octane salt of formula (I), wherein a compound H-Ar is reacted with 1-R¹-4-fluor-1,4-diazonia bicyclo [2.2.2] octane-bis-anion in the presence of a catalyst to yield a compound of the formula (I) 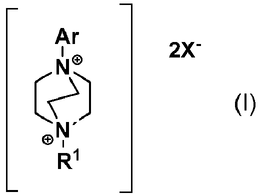 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or heteroaryl-C₆ to C₂₀-alkyl group, each group being optionally substituted, and
X⁻ is an anion.

In one embodiment of the process for preparing a polymer comprising at least one Ar-N-R¹-N'-diazoniabicyclo-[2.2.2]octane salt unit of formula (I), wherein at least one Ar is bonded directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof, a polymer having said at least one mono- or polycyclic aromatic or heteroaromatic hydrocarbon Ar, optionally being substituted by one or more substituents, bonded to the polymer chain is reacted with 1-R¹-4-fluor-1,4-diazoniabicyclo [2.2.2] octane-bis-anion in the presence of a catalyst to yield a polymer comprising at least one Ar-N-R¹-N'-diazoniabicyclo-[2.2.2]octane salt unit of formula (I), wherein Ar, R¹ and X have the meanings as given above.

In a further embodiment of the inventive process for preparing a polymer comprising at least two Ar-N-R¹-N'-diazoniabicyclo-[2.2.2]octane salt units of formula (I), wherein at least two Ar are bonded directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof, at least two Ar-N-R¹-N'-diazoniabicyclo[2.2.2]octane salt units of formula (I), 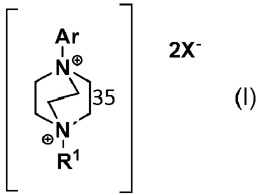 wherein

Ar is a mono- or polycyclic aromatic or heteroaromatic hydrocarbon, optionally being substituted by one or more substituents and Ar is having a substituent R² with a polymerizable group, are reacted, optionally in the presence of further polymerizable compounds, to polymerize via the polymerizable group, wherein Ar, R¹ and X have the meanings as given above. In such case, Ar might have an aryl or heteroaryl substituted olefin structure such as of styrene and the polymerization might be carried out in the presence of further olefinic compounds such as divinyl benzene.

In the inventive process, the catalyst may preferably be a single electron redox catalyst which is used alone or in a mixture of two or more, preferably selected from (i) a combination of Pd complex A and Ru(bipy)₃(PF₆)₂ (bipy = 2,2'-bipyridine), or (ii) a Cu(II) salt, such as CuBr₂.

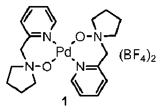

Thus, the present invention also comprises the use of a compound 1-R¹-4-fluor-1,4-diazonia bicyclo [2.2.2] octane-bis-anion of the formula (III) 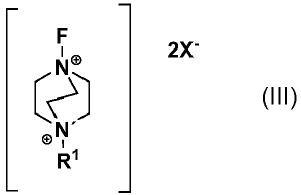 for a process for charge transfer directed radical substitution of aromatic or hetero aromatic compounds, preferably in para-position of a monosubstituted aryl compound, wherein R¹ and X have the meaning as given before.

The compound of the formula (I) can be converted into an Ar-piperazine. Thus, the invention is also directed to said process for preparing an Ar-piperazine as represented by the following formula (II): 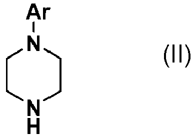 wherein a Ar-N-R¹-N'-diazoniabicyclo[2.2.2]octane salt of formula (I) 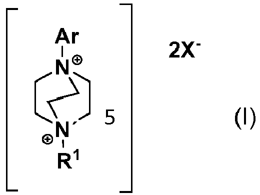 is reacted with a reducing agent, preferably sodium thiosulfate in an aqueous solution, and preferably at an elevated temperature, to yield the Ar-piperazine as target compound, wherein Ar, R¹ and X have the meanings as given above.

The invention is also directed to said Ar-piperazine as represented by the following formula (II): 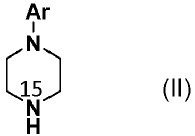 wherein Ar has the meaning as given above, which is obtainable according to the process described before.

The invention is also directed to the use of a compound of the formula (III) 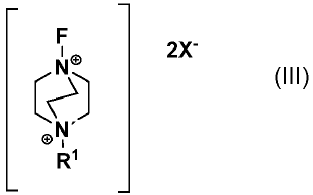 for a process for charge transfer directed radical substitution of aromatic or hetero aromatic compounds, wherein R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or C₅ to C₂₀ heteroaryl-C₆ to C₂₀-alkyl group, each group being optionally substituted, and X- is an anion.

The compounds of formula (I) and (II) can be used for preparing functionalized polymers as detailed further below.

The substituents which are useful for the compounds of the present invention are detailed as follows.

A substituent or heterosubstituent as defined according to the invention can be selected from OH, F, Cl, Br, I, CN, NO₂, SO₃H a monohalogenomethyl group, a dihalogenomethyl group, a trihalogenomethyl group, CF(CF3)₂, SF₅, amine bound through N atom, -O-alkyl (alkoxy), -O-aryl, -O-SiR^{S}₃, S-R^{S}, S(O)-R^{S}, S(O)₂-R^{S}, COOH, CO₂-R^{S}, -BR^{s}₂, -PR^{S}₂,-OPR^{S}₂, amide, bound through C or N atom, formyl group, C(O)-R^{S}, COOM, where M may be a metal such as Na or K. R^{S} may be, independently from each other, the same or different and may be each an aliphatic, heteroaliphatic, aromatic or heteroaromatic group, each optionally being further substituted by one or more heterosubstituents, aliphatic, heteroaliphatic, aromatic or heteroaromatic groups.

Aliphatic hydrocarbons including alkyl, alkenyl and alkinyl may comprise straight-chain, branched and cyclic hydrocarbons.

Heteroaliphatic is a hydrocarbon including alkyl, alkenyl and alkinyl which may comprise straight-chain, branched and cyclic hydrocarbons with one or more carbon atoms substituted with a heteroatom.

In more detail, C₁-C₂₀-Alkyl can be straight chain or branched and has 1, 2, 3, 4, 5, 6, 7, 8, 9, 10, 11, 12, 13, 14, 15, 16, 17, 18, 19 or 20 carbon atoms. Alkyl might be C₁-C₆-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl. Substituted alkyl groups are trifluoromethyl, pentafluoroethyl and 1,1,1-trifluoroethyl.

Cycloalkyl might be cyclopropyl, cyclobutyl, cyclopentyl, cyclohexyl or cycloheptyl. Alkenyl might be C₂-C₂₀ alkenyl. Alkinyl might be C₂-C₂₀ alkinyl.

Said unsaturated alkenyl- or alkinyl groups can be used for linking the inventive compounds to a carrier such as a polymer to serve for an immobilized catalyst.

Halogen is F, Cl, Br or I.

Alkoxy is preferably C₂-C₁₀ alkoxy such as methoxy, ethoxy, propoxy, *tert-*butoxy etc.

C₃-C₈-Heteroalkyl having one or more heteroatoms selected from among N, O and S is preferably 2,3-dihydro-2-, -3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or - 5-pyrrolyl, 2,5-dihydro-1-, -2-, -3-, -4- or -5-pyrrolyl, 1-, 2- or 3-pyrrolidinyl, tetrahydro-1-,-2- or -4-imidazolyl, 2,3-dihydro-1-, -2-, -3-, -4- or -5-pyrazolyl, tetrahydro-1-, -3- or -4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4- or -5-yl, hexahydro-1-, -3- or -4-pyridazinyl, hexahydro-1-, -2-, -4- or-5-pyrimidinyl, 1-, 2- or 3-piperazinyl, 1,2,3,4-tetrahydro-1-, -2-, -3-, -4-, -5-, -6-, - 7- or -8-quinolyl, 1,2,3,4-tetrahydro-1-, -2-, -3-, -4-, -5-, -6-, -7- or -8-isoquinolyl, 2-, 3-, 5-, 6-, 7- or 8-3,4-dihydro-2H-benzo-1,4-oxazinyl.

Optionally substituted means unsubstituted or monosubstituted, disubstituted, trisubstituted, tetrasubstituted, pentasubstituted, or even further substituted for each hydrogen on the hydrocarbon.

Aryl might be phenyl, naphthyl, anthracenyl, phenanthryl or biphenyl, or further polycyclic aromatic hydrocarbons including fullerenes such as C₆₀, C₇₀, C₇₆, C₈₀, C₈₂, C₈₄, C₈₆, C₉₀ und C₉₄.fullerenes

Arylalkyl might be benzyl.

Heteroaryl may have one or more heteroatoms selected from among N, O and S and is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, also preferably 1,2,3-triazol-1-, - 4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or 4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6-or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7- or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

The inventive process is further illustrated in the following exemplary reaction scheme 1:

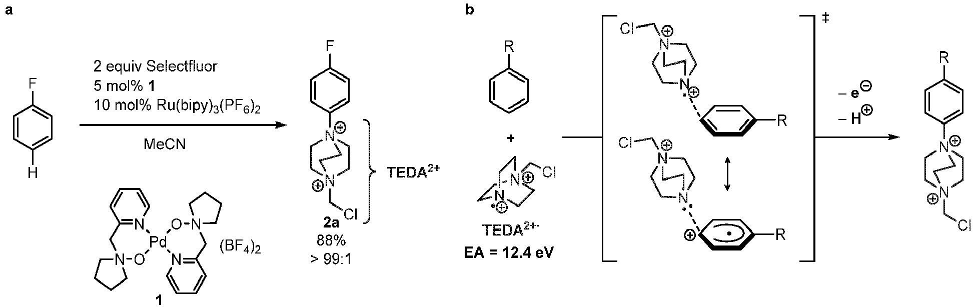

As shown, the charge transfer directed aromatic substitution is a conversion of fluorobenzene to the corresponding Ar-TEDA compound **2a**. The positional selectivity of TEDA²⁺ substitution is due to the stabilizing effect of arene-to-radical charge transfer in the transition state of addition (EA = Electron Affinity, refers to gas phase adiabatic electron affinity calculated by DFT).

The catalyst is usually a single electron redox catalyst used alone or in a mixture of two or more. Examples of the catalyst are Cu^{II}X and Ru^{II} X₂ catalysts, such as Ru(bipy)₃(PF₆)₂ , preferably in combination with a Pd catalyst of the formula:

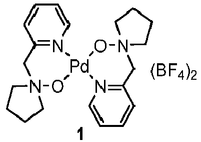

The Aryl-TEDA compounds are doubly cationic compounds, and similar cationic compounds are formed as byproducts of the reaction, including H-TEDA²⁺ and TEDA⁺.

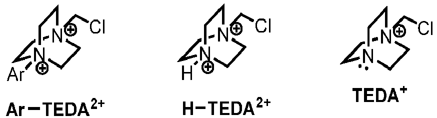

Therefore, it might be difficult to isolate the Aryl-TEDA products from the reaction mixture. The inventors have found that performing the reaction with an excess (at least five equivalents) of the arene substrate minimizes formation of H-TEDA²⁺ and TEDA⁺ byproducts as shown exemplarily in the following scheme:

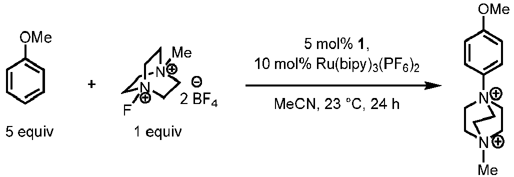

Upon reaction completion, evaporation of the solvent, followed by trituration of the residue with CH₂Cl₂/methanol mixtures to remove the excess arene, and the palladium and ruthenium catalysts, results in material that is sufficiently clean for characterization, albeit still contaminated to varying degrees with H-TEDA²⁺ and TEDA⁺.

Therefore, the inventors have performed the Ar-TEDA formation reactions twice for each of the Ar-TEDA products **2a**-**g** (in the experimental part below): once utilizing the arene as limiting reagent (with yield determined by NMR integration relative to an internal standard), and once with excess arene (for characterization purposes). For Ar-TEDA compounds synthesized through the use of excess arene, yields of Ar-TEDA, H-TEDA²⁺, and TEDA⁺ are calculated relative to Selectfluor, and were determined via NMR analysis of a known amount of the mixture by integrating against an internal standard.

The Ar-TEDA products may be isolated from H-TEDA²⁺, and TEDA⁺ by repeated recrystallization. The Ar-TEDA product derived from fluorenone (2f) was isolated in this way and characterized as a pure compound.

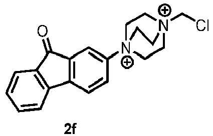

### Experimental Results and Discussion:

### Charge Transfer Directed Radical Substitution:

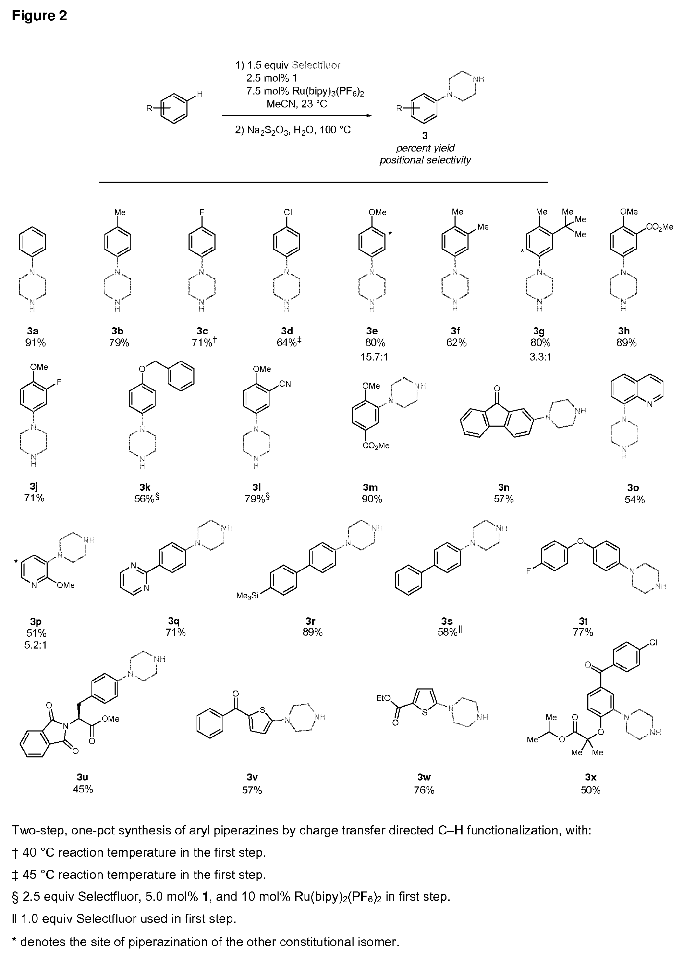

The doubly cationic radical TEDA^{2+•}, derived from single electron reduction of Selectfluor, is capable of engaging in radical aromatic substitution to yield *N*-aryl-*N'*-chloromethyl-diazoniabicyclo[2.2.2]octane salts, which the inventors have termed Ar-TEDA compounds (Figure 2).

The reaction is exemplarily enabled by a dual catalyst combination: Pd catalyst 1 above, which the inventors have introduced in a previous report, and Ru(bipy)₃(PF₆)₂ (Figure 2a). Photoirradiation is not required for reaction, which works equally well when shielded from light. For most arenes only one of the possible positional isomers of the Ar-TEDA product is observed as judged by nuclear magnetic resonance spectroscopy; fluorobenzene, for example, yields the para substituted product in >99:1 positional selectivity. All monosubstituted arenes tested give the *para* substituted product as the only significant isomer. Disubstituted arenes and some heteroarenes likewise undergo clean substitution at the position *para* to the group with the strongest directing effect. Thus, the synthesis of Ar-TEDA compounds described here constitutes a general non-chelation-assisted C-H functionalization reaction, with the arene as the limiting reagent, with nearly exclusive positional selectivity across a broad range of substitution patterns.

As stated by the inventors, the TEDA^{2+•} radical is an electrophilic radical, with an electron affinity of 12.4 eV, calculated by DFT. The high electron affinity of TEDA^{2+•} should favor a large contribution of charge-transfer in the transition state of addition (Figure 2B), which in turn leads to high selectivity for aromatic substitution at the position from which charge transfer is the greatest. Therefore, a predictive tool for the positional selectivity of the reaction would be a metric which indicates the greatest extent of charge transfer that can be expected upon attack at a given position. The inventors found Fukui nucleophilicity indices to be well-suited to this purpose. The Fukui nucleophilicity index of an atom, determined by simple quantum chemical calculations, is a measure of how readily electron density is transferred to an incoming electrophilic species attacking at the relevant atom. Fukui indices are especially convenient as a predictive tool because the Fukui index for all atoms in a given molecule are determined by a pair of simple calculations on the arene itself; there is no need to map the potential energy surface of the reaction by computing the transition states of various pathways.

The high degree of positional selectivity the inventors report here is unusual, especially in the context of radical aromatic substitution. A reason may be a lack of studies of substitution reactions by radicals of high electron affinity. While the TEDA^{2+•} radical dication has been proposed as an intermediate in recently reported aliphatic C-H oxidation methodologies utilizing Selectfluor, to the inventors' knowledge addition of this radical to unsaturated systems has not been investigated. However, aromatic substitution reactions of most neutral radicals are known to proceed with low selectivity.

Positive charge increases electron affinity, and based on the inventors' findings and proposal, positively charged radicals result in more selective arene substitution reactions. The inventors have discovered that, for sufficiently electrophilic radicals, charge transfer in the transition state of addition can lead to high selectivity for the *para* position over *ortho;* the inventors have rationalized the phenomenon in terms of charge transfer in the transition state, and have introduced Fukui indices as a tool for predicting the site of substitution.

The general applicability of the charge-transfer directed concept will depend on whether other radicals of comparable electron affinity to TEDA^{2+•} can be designed. The uncommonly high electron affinity of TEDA^{2+•} is due to its two positive charges; doubly cationic organic radicals are rare, presumably because there has been a lack of generally appreciated applications and because strategies for accessing them are unexplored. The inventors anticipate that the correlation between electron affinity and positional selectivity described herein will stimulate research in high electron affinity radicals due to their potential to address the longstanding challenge of positional selectivity in C-H functionalization.

The inventors furthermore note that radicals of electron affinity comparable to TEDA^{2+•} need not in principle be based on cationic aminium radicals. For example, DFT calculations indicate that alkoxyl radicals exhibit a similar trend with increasing positive charge, though the septet oxygen atom itself lacks a formal charge (Supplementary Figure S11). Thus, in principle, highly electrophilic radicals could be designed for the installation of a variety of functional groups, not just C-N bonds.

### Application to the synthesis of aryl piperazines:

As one synthetic application of the charge transfer directed radical substitution concept, the inventors have developed a two-step, one-pot synthesis of aryl piperazines from the corresponding aryl C-H compounds (Figure 2).

The procedure involves reduction of the Aryl-TEDA compounds by sodium thiosulfate, which converts the TEDA moiety into a piperazine heterocycle. Piperazines are a common motif in pharmaceuticals and materials; they constitute the third most common heterocycle present in the small molecule pharmaceuticals listed in the FDA Orange Book. Aryl piperazines are commonly synthesized by Buchwald-Hartwig cross coupling reactions of aryl electrophiles with piperazine derivatives. The direct synthesis of aryl piperazines reported here is advantageous because it does not require a pre-functionalized substrate, such as an aryl halide. Importantly, this advantage relies on the high and predictable positional selectivity of the reaction, which enables the high-yield synthesis of a single desired positional isomer. The reaction is operationally simple, and can be performed under air with commercial-quality solvent. Furthermore, the piperazine moiety is obtained with an unprotected secondary amine, ready for subsequent manipulation.

A variety of arenes, including 5- and 6-membered heteroarenes, undergo piperazination. Generally, attack of TEDA^{2+•} *ortho* to substituents is unfavorable, and occurs only for arenes in which the preferred *para* position for substitution is blocked by a group which cannot undergo *ipso* substitution; this observation can be applied to block piperazination of certain positions, or even entire arene rings, as in substrates **3r** and **3t.** Product **3g** demonstrates the limits of the positional selectivity of the reaction: the two substituents in 2-methyl-tert-butylbenzene differ only slightly in their electron-donating ability, and the product was isolated as a 3.3:1 mixture of isomeric products. Although TEDA^{2+•} is known to engage in sp³ C-H bond cleavage, the inventors have observed no evidence of such side-reactions in the inventors' investigations, despite the fact that several substrates contain weak C-H bonds adjacent to aromatic rings (e.g., **3f**) or ether oxygen atoms (e.g., **3e, 3k**); addition of TEDA^{2+•} to the unsaturated aromatic system outcompetes C-H bond cleavage.

For most substrates, nearly full conversion to the Ar-TEDA compound is observed, and in several cases the yield of the piperazine following the thiosulfate-mediated stage is lower. For example, the anti-cholesterol drug Fenofibrate undergoes Ar-TEDA formation in 88% yield, but upon treatment with sodium thiosulfate at 100 °C the yield of piperazine **3x** is 51%. The Ar-TEDA formation reaction exhibits significant functional group tolerance, despite the highly reactive and electrophilic nature of the TEDA^{2+•} radical intermediate; for most substrates in Table 1, the majority of mass balance is lost in the piperazine formation step, not the Ar-TEDA formation step.

The inventors have shown that the doubly cationic nitrogen-based radical TEDA^{2+•} undergoes radical substitution with arenes with higher positional selectivity than any conventional methodology for arene substitution. Thus, the inventors put forth a previously underappreciated rationale to explain and predict positional selectivities in charge transfer directed radical aromatic substitution: high selectivity is achieved through a high degree of charge-transfer in the transition state of addition. This charge transfer effect is maximized for radicals with high electron affinity. The inventors' results can rationalize why known electrophilic radical substitution reactions of neutral radicals are typically not selective, and more importantly, they provide a framework to guide the design of new, selective arene substitution chemistry.

The inventors have extended the inventive concept to further applications. A further way to take advantage of the inventive reaction is to apply the reaction conditions to polymers, such as simple commercially available polystyrenes and poly(styrene-co-divinylbenzene), to access functionalized polymers (Scheme 22).

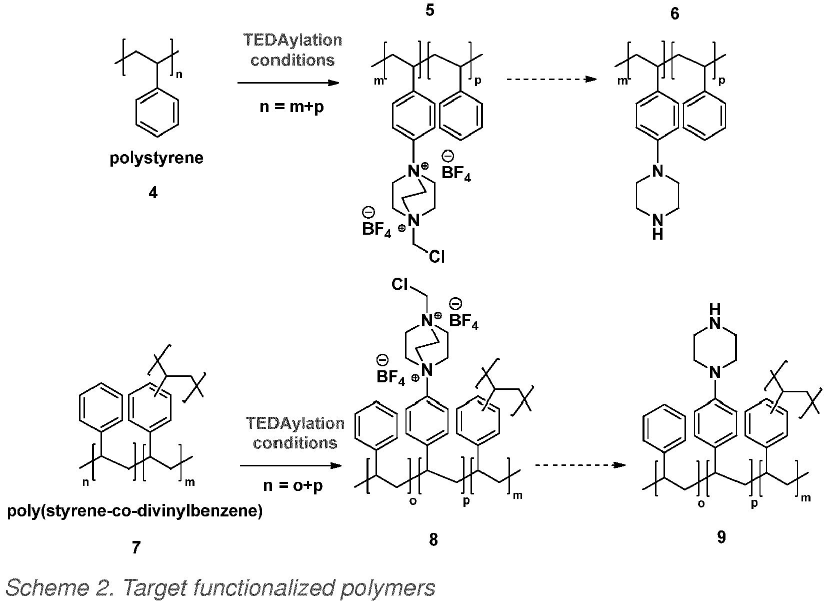

These polymers could be also synthesized from the polymerization reaction of styrene monomers and pre-functionalized styrene monomers, in the presence (or not) of divinylbenzene (dvb) (Scheme 3).

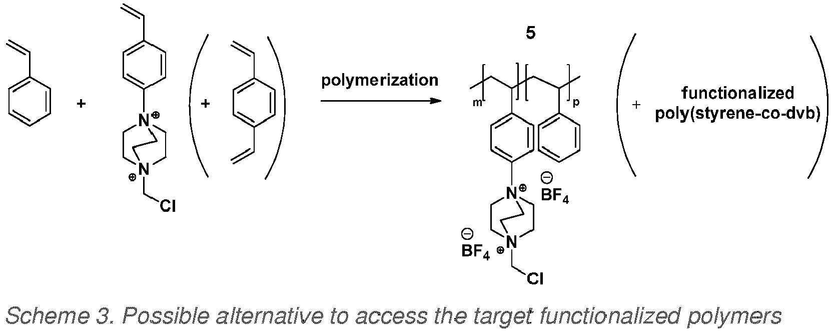

The introduction of such moiety could provide interesting properties to the polymers and led to useful applications. Among them, use these polymers as ion-exchange resins is a choice, considering already existing charged polymers used in the everyday life (see below). Moreover, the polymers could be also used as supports to immobilize reagents or catalysts, especially after formation of the piperazine.

### Ion-exchange resin

Usually, an ion-exchange resin or ion-exchange polymer is an insoluble matrix (or support structure) normally in the form of small beads fabricated from an organic polymer substrate. The beads are typically porous, providing a high surface area. The trapping of ions occurs with the accompanying releasing of other ions; thus the process is called ion-exchange. In the present case, the obtained crosslinked polymers can be considered as strongly basic anion exchange resins, able to trap anionic species due to the presence of quaternary amines. Even if strongly acidic resins are much more employed as purification/separation agents (such as polystyrene sulfonate), an interesting example of strongly basic resin is the cholestyramine, a commercialized drug under the name of Questran, used as bile acid sequestrant (Scheme 4 ).

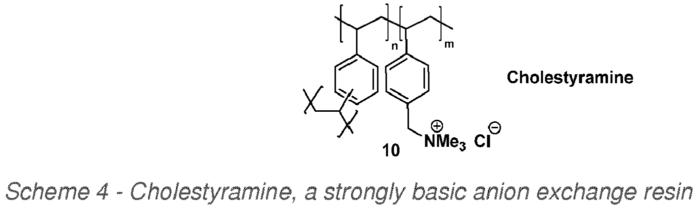

This polymer is a Dowex 1-X2 resin (Dow Chemicals), which is a functionalized poly(styrene-co-divinylbenzene) bearing a quaternary amine at the para-position of the aromatic rings. Its synthesis is accomplished through the chloromethylation and subsequent quaternarization with trimethylamine of a divinylbenzene cross-linked styrene polymer (5).

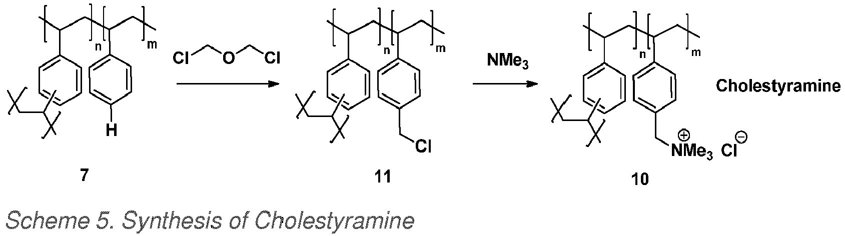

The inventor's approach leads to a synthesis of such a resin by one step and though a metal-catalyzed reaction. Indeed, the TEDAylation of poly(styrene-co-divinylbenzene) could lead in one step to the resin **5** (Scheme 2), which possesses two quaternary amine; therefore it would be reasonable to think that this new resin could be, at best, two-times more efficient than the cholestyramine. The efficiency relies on the accessibility of the ammonium salt inside the beads, depending itself on the porosity of the resin, but also on the number of TEDA moieties grafted on the copolymer. Since its discovery, the role of the cholestyramine has decreased in importance because of the introduction of statins, but others applications remain, related to the elimination of bile acids, elimination of drugs or treatment of diarrheas.

Another utility of ion-exchange resin is illustrated by Dowex 2, another strongly basic resin possessing a quaternary ammonium salt, applied in a plant that processed 2,500,000 gallons of water per day, reducing the silica level to < 0.05 ppm.

Alternatively, the linear polymer **6** obtained after conversion of the TEDA moiety into piperazine might also be useful, by comparison with the polyethylenimine **12** employed as additive in detergents or adhesives (Scheme 6).

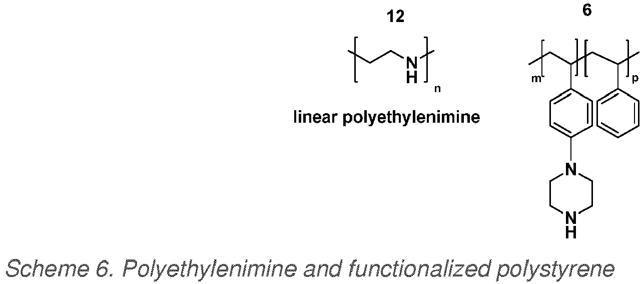

Moreover, the linear polymer **6** can also be used as decontaminant, as the Atrazine, a widely applied chlorotriazine herbicide which has been found in water in agricultural regions, is removed from water due to its toxicity via an amine-functionalized polystyrene (Scheme 7). The Atrazine is removed from water by covalently binding.

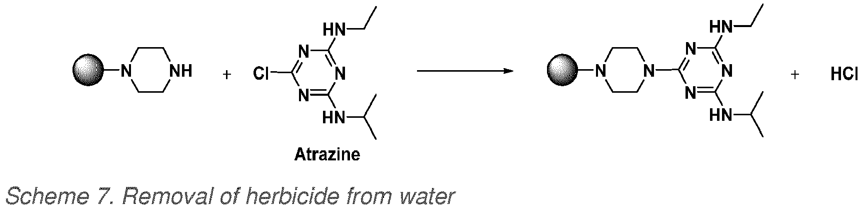

### Immobilization of reagents and catalysts

Catalysts and reagents immobilized on functionalized polystyrenes have been reported and used in a wide range of typical transformations such as oxidation, reduction and C-C bound formations. The main benefits are due to the ease of physical separation of the polymer and its bound component from the reaction mixture, the ease of recycling (especially with expensive catalysts and ligands), and the simplification of handling a range of toxic or odorous materials.

The two most common methods to immobilize a reagent or a catalyst are achieved through ion-pairing or covalent binding. In the former mode of attachment, two approaches can be employed: the first one involves the grafting of the catalyst or reagent on the prederivatized support of the present invention, but without prederivatization, and the second one relies on the copolymerization reaction of the active specie with styrene and divinylbenzene (Scheme 3).

Linear polystyrenes (LPS) are used as soluble support for organic synthesis, as well as polyethyleneglycol. This technic proved to be useful for the preparation of libraries of small molecules. Most of the time, polystyrenes functionalized by a chloromethyl moiety are employed, first reacting via nucleophilic substitution in presence of appropriate nucleophiles as described below in the synthesis of prostaglandin (Scheme 8).

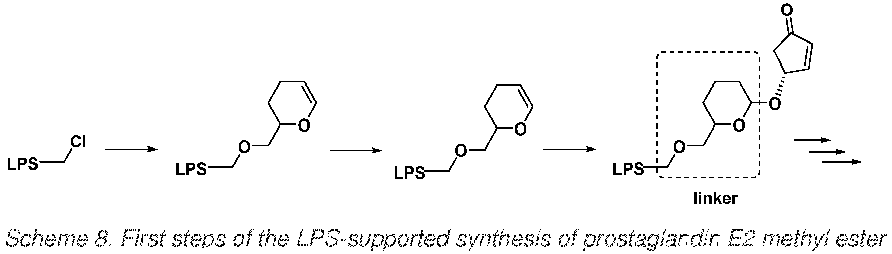

According to the present invention, TEDAylation of LPS followed by the formation of the piperazine enables the access to suitable polymers for soluble polymer-supported synthesis.

Cross-linked poly(styrene-co-divinylbenzene) resins are also widely used as functionalized polymers because of their stability, high loading capacity and good swelling characteristics. The immobilization of counterions proved to be useful in oxidation of alcohols (Schem 9), reduction of ketones, as well as halogenation reactions and C-C

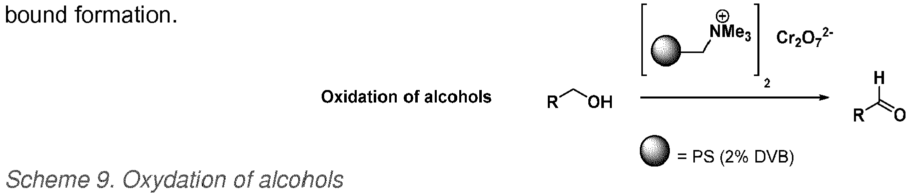

In that context, we can envisage the immobilization of appropriate counterions with the polymers 5 and 8 to achieve the previously described chemical transformations.

### Functionalization of fullerenes

In medicinal chemistry, C₆₀ has shown activity against HIV and microorganisms, but the major drawback of fullerene is its poor solubility or insolubility in protic and polar aprotic organic solvents. This limitation can be bypassed by functionalization. In that context, works have been realized with amphiphilic fulleropyrrolidine derivatives, obtained via [3+2] cycloaddition with azomethine ylides, the most successful method for the functionalization of fullerenes. Introduction of positive charges (ammonium) increased the solubility of the molecules but also brought interesting properties such as antimycobacterial activity and ability to self-organize in solution at the air-water interface and onto surfaces (amphipilic derivatives). The more positively charged the fullerene derivative is, the more soluble in aqueous media or physiological media it becomes (Scheme 10, **13**). This also increased the activity on HIV-1 and -2 strains.

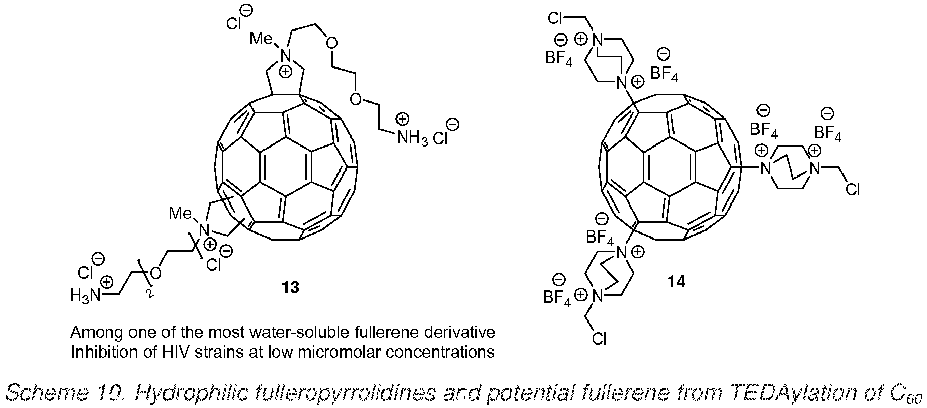

In that context, grafting one or more TEDA moieties on a C₆₀ is promising (Scheme 10, 14), in terms of the solubility and the behavior of such amphiphilic species in different medias, and its therapeutic activity (HIV, etc...). Thus, grafting one or more TEDA moieties on a C₆₀ fullerene can enhance typical aqueous or biological applications, such as photocleveage, drug and gene delivery, enzyme inhibition or antibiological activity. Thus, the present invention also comprises such kind of fullerenes including C60, C70, C76, C80, C82, C84, C86, C90 und C94.fullerenes as AR in the inventive TEDAylated compounds and their use as therapeuticals.

### Functionalization of copolymers

The styrene-butadiene is a synthetic rubber (SBR), whose its physical properties depend on the ratio between styrene and butadiene monomers.

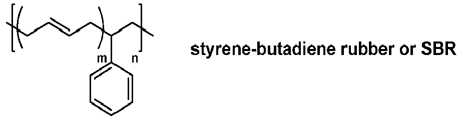

This polymer is widely used in pneumatic tires, but also in coated papers allowing the binding of pigmented coatings. That property could be enhanced in the presence of TEDA moieties on the polymer.

The inventive process can also be used for preparing TEDAylated aromatic compounds for pharmaceutical or medical applications. A possible application is as anti-fouling coatings. Microbial contamination and the associated risk of infection is one of the most serious complications in food industries, hospitals, and community settings. The attachment of bacteria to a surface leads to subsequent colonization resulting in the formation of a biofilm. To prevent such contamination, different types of antimicrobial coatings have been developed. Among them, a polycation-based coating, acting as a non-released-based antimicrobial system, represents a novel and efficient approach. Indeed, quaternized polymers, such as bearing ammonium moieties, can be bactericidal through a contact mechanism. In this context, grafting TEDA-functionalized polymers on sensitive surfaces which need to be protected could be another useful application of the inventive reaction.

### Experimental Part

### Materials and Methods

All air- and moisture-insensitive reactions were carried out under an ambient atmosphere, magnetically stirred, and monitored by thin layer chromatography (TLC) using EMD TLC plates pre-coated with 250 µm thickness silica gel 60 F254 plates and visualized by fluorescence quenching under UV light. Flash chromatography was performed on Dynamic Adsorbents Silica Gel 40-63 µm particle size using a forced flow of eluent at 0.3-0.5 bar pressure.³² All air- and moisture-sensitive manipulations were performed using oven-dried glassware, including standard Schlenk and glovebox techniques under an atmosphere of nitrogen. Acetonitrile and acetonitrile-*d*₃ were dried over P₂O₅ and vacuum-distilled. MeOH was degassed at -30 °C under dynamic vacuum (10⁻⁴ Torr) for one hour and stored over 3A sieves. All chemicals were used as received. All deuterated solvents were purchased from Cambridge Isotope Laboratories. NMR spectra were recorded on either a Varian 3Unity/Inova 600 spectrometer operating at 600 MHz for ¹H acquisitions, a Varian Unity/Inova 500 spectrometer operating at 500 MHz and 125 MHz for ¹H and ¹³C acquisitions, respectively, or Varian Mercury 400 spectrometer operating at 375 MHz and 101 MHz for ¹⁹F and ¹³C acquisitions, respectively. Chemical shifts are reported in ppm with the solvent resonance as the internal standard (¹H: CDCl₃, δ 7.26; (CD₃)₂SO, δ 2.50 (CD₃)₂CO, CD₃CN, δ 1.94), (¹³C: CDCl₃, δ 77.16; (CD₃)₂SO, δ 39.52; (CD₃)₂CO, δ 29.84; CD₃CN, δ 1.32),³³ or added 3-nitrofluorobenzene (-112.0 ppm) for ¹⁹F spectra. Signals are listed in ppm, and multiplicity identified as s = singlet, br = broad, d = doublet, t = triplet, q = quartet, quin = quintet, sep = septet, m = multiplet; coupling constants in Hz; integration. High-resolution mass spectra were obtained using an Agilent ESI-TOF (6210) mass spectrometer or a Bruker q-TOF Maxis Impact mass spectrometer. Concentration under reduced pressure was performed by rotary evaporation at 25-30 °C at appropriate pressure. Purified compounds were further dried under high vacuum (0.01-0.05 Torr). Yields refer to purified and spectroscopically pure compounds, unless otherwise noted.

### Procedure for Preparation of Palladium Complex 1

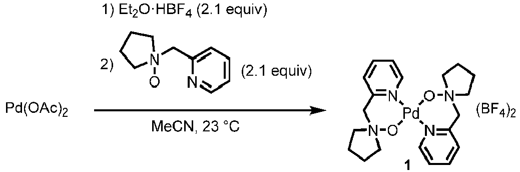

A flame-dried, 250 mL 2-neck flask under nitrogen was charged with Pd(OAc)₂ (5.00 g, 22.3 mmol, 1.00 equiv), and the flask was evacuated and refilled with N₂. Through a septum was added dry acetonitrile (50 mL, Aldrich Sure/Seal™), followed by Et₂O-HBF₄ (6.4 mL, 47. mmol, 2.1 equiv). The resulting suspension was stirred at 23 °C for 30 min, after which 1-(pyridine-2-ylmethyl)pyrrolidine 1-oxide (8.334 g, 46.77 mmol, 2.10 equiv) was added as a solution in dry acetonitrile (40 mL, Aldrich Sure/Seal™). The resulting mixture was stirred for 1 hr, after which the reaction mixture was diluted with 100 mL acetonitrile to dissolve the precipitated product, and the solution was filtered through celite and then concentrated by rotary evaporation. The resulting brown solid was triturated with dichloromethane (40 mL) with sonication. The product was collected by filtration on a glass frit, then washed with dichloromethane (40 mL) followed by tetrahydrofuran (40 mL), then allowed to dry on the frit with applied suction to yield 10.61 g of a yellow powder (16.66 mmol, 75%).
NMR Spectroscopy: ¹H NMR (600 MHz, DMSO, 23 °C, δ): 8.50 (dd, *J =* 5.8, 1.2 Hz, 2H), 8.30 (ddd, *J* = *7.7,* 7.7, 1.5 Hz, 2H), 7.86 (ddd, *J =* 7.5, 5.8, 1.4 Hz, 2H), 7.79 (dd, *J =* 7.8, 1.2 Hz, 2H), 5.35 (s, 4H), 3.56-3.46 (m, 4H), 3.45-3.36 (m, 4H), 2.26-2.15 (m, 4H), 2.10-1.99 (m, 4H). ¹³C NMR (125 MHz, DMSO, 23 °C, δ): 149.2, 148.2, 142.0, 128.2, 126.5, 70.1, 67.2, 21.3.
Mass spectrometry: HRMS-FIA(m/z) calcd for C₂₀H₂₈N₄O₂Pd [M]²⁺, 231.0622; found, 231.0632.
Anal. Calcd for C₂₀H₂₈B₂F₈N₄O₂Pd: C, 37.74; H, 4.43; N, 8.80. Found: C, 37.83; H, 4.14; N, 9.04.

### 1-(Chloromethyl)-4-(4-fluorophenyl)-1A-diazabicyclor2.2.2]Octane²⁺ (2a)

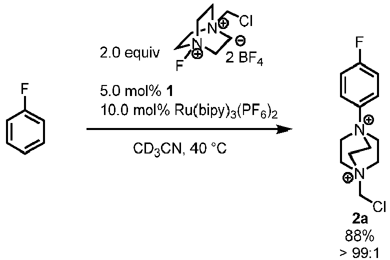

A 4 mL vial was charged with Selectfluor (70.9 mg, 0.200 mmol, 2.00 equiv), 1 (3.2 mg, 5.0 µmol, 5.0 mol%), and Ru(bipy)₃(PF₆)₂ (8.6 mg, 10 µmol, 10. mol%), and 0.50 mL CD₃CN, and finally fluorobenzene (9.4 µL, 0.10 mmol, 1.0 equiv). The vial was sealed and the mixture stirred at 40 °C for 36 h. The reaction mixture was diluted with 0.25 mL CD₃CN and filtered through a 0.22 µm PVDF syringe filter, and an additional 0.25 mL CD₃CN was washed through the filter to elute any remaining soluble material. The solution was analyzed by ¹⁹F NMR: The product mixture of the reaction to form **2a** was analyzed by ¹⁹F NMR in several different solvents in order to rule out coincidental overlap with any peaks corresponding to constitutional isomers of **2a**. The analysis was carried out by evaporating the acetonitrile solvent from the reaction mixtures and dissolving the residue in 5:1 CD₃CN:C₆D₆, *d6*-DMSO, and *d₅*-pyridine, respectively. In each of these cases, only one aryl fluoride signal was observed by ¹⁹F NMR:

### Formation of 2a in the absence of light

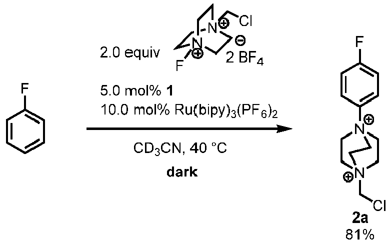

A 4 mL vial, completely covered with aluminum foil, was charged with Selectfluor (70.9 mg, 0.200 mmol, 2.00 equiv), 1 (3.2 mg, 5.0 µmol, 5.0 mol%), and Ru(bipy)₃(PF₆)₂ (8.6 mg, 10 µmol, 10. mol%), and 0.50 mL CD₃CN, and finally fluorobenzene (9.4 µL, 0.10 mmol, 1.0 equiv). The vial was sealed and the mixture stirred in the dark at 40 °C for 24 h, after which 2.0 µL of 3-fluoronitrobenzene was added as an internal standard. The reaction mixture was diluted with 0.50 mL CD₃CN, and passed through a 0.22 µm PVDF syringe filter. The resulting solution was analyzed by ¹⁹F NMR, and comparison of the Ar-F peak of the product (-110 ppm) with that of the internal standard (-112 ppm) revealed a yield of 81% of **2a**.

### 1-(Chloromethyl)-4-(4-chlorophenyl)-1,4-diazabicyclo[2.2.2]octane²⁺ (2b)

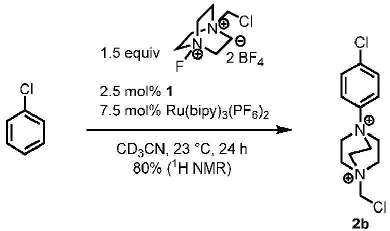

Palladium complex **1** (10.3 mg, 16.2 µmol) and Ru(bipy)₃(PF₆)₂ (41.9 mg, 48.8 µmol) were dissolved in *d₃*-acetonitrile (3.25 mL) to afford a stock solution. A 4 mL vial was charged with Selectfluor (52.2 mg, 0.147 mmol, 1.50 equiv). The stock solution (491. µL) containing 1 (2.45 µmol, 2.50 mol%) and Ru(bipy)₃(PF₆)₂ (7.37 µmol, 7.50 mol%) was added, followed by chlorobenzene (10.0 µL 98.2 µmol, 1.00 equiv, c = 0.20 M) via syringe. After stirring at 23 °C for 24 h, the reaction mixture was diluted with *d₃*-acetonitrile (3.0 mL). The yield of **2b** was determined via ¹H NMR spectroscopy using ethyl acetate (5.0 µL, 51. µmol) as an internal standard. Comparison of the integral of the peak of 2b at 7.78-7.81 ppm (aromatic C-H, 2H) with that of the peak of ethyl acetate at 1.20 ppm (CH₃, 3H) revealed an 80% yield **of 2b.**

### 1-(Chloromethyl)-4-(4-methylphenyl)-1,4-diazabicyclo[2.2.2]octane²⁺ (2c)

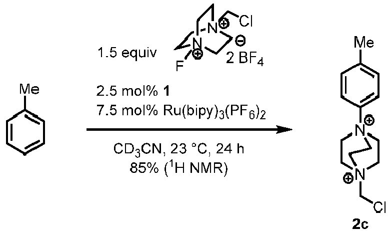

Palladium complex **1** (10.3 mg, 16.2 µmol) and Ru(bipy)₃(PF₆)₂ (41.9 mg, 48.8 µmol) were dissolved in *d₃*-acetonitrile (3.25 mL) to afford a stock solution. A 4 mL vial was charged with Selectfluor (49.9 mg, 0.141 mmol, 1.50 equiv). The stock solution (469. µL) containing **1** (2.34 µmol, 2.50 mol%) and Ru(bipy)₃(PF₆)₂ (7.04 µmol, 7.50 mol%) was added, followed by toluene (10.0 µL, 93.9 µmol, 1.00 equiv, c = 0.20 M) via syringe. After stirring at 23 °C for 24 h, the reaction mixture was diluted with *d₃*-acetonitrile (3.0 mL). The yield of **2c** was determined via ¹H NMR spectroscopy using ethyl acetate (5.0 µL, 51. µmol) as an internal standard. Comparison of the integral of the peak of 2c at 7.67-7.65 ppm (aromatic C-H, 2H) with that of the peak of ethyl acetate at 1.20 ppm (CH₃, 3H) revealed an 85% yield of **2c.**

### 1-(Chloromethyl)-4-(4-methoxyl)henyl)-1,4-diazabicyclo[2.2.2]octane²⁺ (2d)

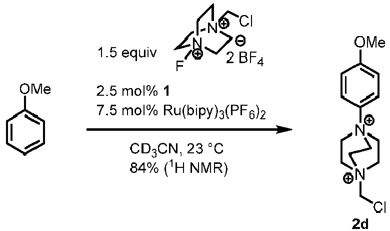

Palladium complex 1 (10.3 mg, 16.2 µmol) and Ru(bipy)₃(PF₆)₂ (41.9 mg, 48.8 µmol) were dissolved in *d₃*-acetonitrile (3.25 mL) to afford a stock solution. A 4 mL vial was charged with Selectfluor (48.9 mg, 0.138 mmol, 1.50 equiv). The stock solution (460. µL, c = 0.200 M) containing **1** (2.34 µmol, 2.50 mol%) and Ru(bipy)₃(PF₆)₂ (7.04 µmol, 7.50 mol%) was added, followed by anisole (10.0 µL, 92.0 µmol, 1.00 equiv) via syringe. After stirring at 23 °C for 24 h, the reaction mixture was diluted with *d₃*-acetonitrile (3.0 mL). The yield of **2d** was determined via ¹H NMR spectroscopy using ethyl acetate (5.0 µL, 51. µmol) as an internal standard. Comparison of the integral of the peak of **2c** at 7.72-7.69 ppm (aromatic C-H, 2H) with that of the peak of ethyl acetate at 1.20 ppm (CH₃, 3H) revealed an 84% yield **of 2d.**

### 1-(Chloromethyl)-4-(3-fluoro-4-methoxyphenyl)-1,4-diazabicyclo[2.2.2]octane²⁺ (2e)

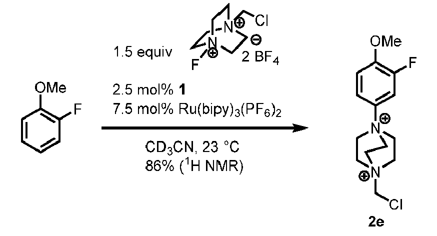

Palladium complex **1** (11.3 mg, 17.8 µmol) and Ru(bipy)₃(PF₆)₂ (45.9 mg, 53.4 µmol) were dissolved in *d₃*-acetonitrile (3.56 mL) to afford a stock solution. A 4 mL vial was charged with Selectfluor (47.3 mg, 0.134 mmol, 1.50 equiv). The stock solution (445. µL, c = 0.200 M) containing **1** (2.23 µmol, 2.50 mol%) and Ru(bipy)₃(PF₆)₂ (6.68 µmol, 7.50 mol%) was added, followed by 2-fluoroanisole (10.0 µL, 89.0 µmol, 1.00 equiv) via syringe. After stirring at 23 °C for 24 h, the reaction mixture was diluted with *d₃*-acetonitrile (2.5 mL). The yield of **2e** was determined via ¹H NMR spectroscopy using ethyl acetate (5.0 µL, 51. µmol) as an internal standard. Comparison of the integral of the peak of 2e at 7.34 ppm (aromatic C-H, 1 H) with that of the peak of ethyl acetate at 1.20 ppm (CH₃, 3H) revealed an 86% yield of **2e.**

### 1-(Chloromethyl)-4-(fluorenon-2-yl)-1,4-diazabicyclo[2.2.2]octane²⁺ (2f)

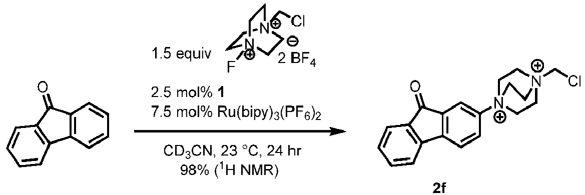

Palladium complex 1 (11.3 mg, 17.8 µmol) and Ru(bipy)₃(PF₆)₂ (45.9 mg, 53.4 µmol) were dissolved in *d₃*-acetonitrile (3.56 mL) to afford a stock solution. A 4 mL vial was charged with Selectfluor (47.3 mg, 0.134 mmol, 1.50 equiv) and fluorenone (16.0 mg, 89.0 µmol, 1.00 equiv). The stock solution (445. µL, c = 0.200 M) containing 1 (2.23 µmol, 2.50 mol%) and Ru(bipy)₃(PF₆)₂ (6.68 µmol, 7.50 mol%) was added via syringe. After stirring at 23 °C for 24 h, the reaction mixture was diluted with *d₃*-acetonitrile (2.0 mL). The yield of **2f** was determined via ¹H NMR spectroscopy using ethyl acetate (5.0 µL, 51. µmol) as an internal standard. Comparison of the integral of the peak of **2f** at 7.94-7.93 ppm (aromatic C-H, 1 H) with that of the peak of ethyl acetate at 1.20 ppm (CH₃, 3H) revealed an 98% yield of **2f.**

### 1-(Chloromethyl)-4-(2-methoxy-5-(methoxycarbonyl)phenyl)-1,4-diazabicyclo[2.2.2]Octane²⁺ (2g)

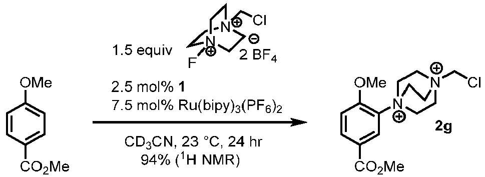

Palladium complex 1 (10.3 mg, 16.2 µmol) and Ru(bipy)₃(PF₆)₂ (41.9 mg, 48.8 µmol) were dissolved in *d₃*-acetonitrile (3.25 mL) to afford a stock solution. A 4 mL vial was charged with Selectfluor (53.1 mg, 0.150 mmol, 1.50 equiv) and methyl 4-methoxybenzoate (16.6 mg, 100. µmol, 1.00 equiv). The stock solution (500. µL, c = 0.200 M) containing 1 (2.50 µmol, 2.50 mol%) and Ru(bipy)₃(PF₆)₂ (7.50 µmol, 7.50 mol%) was added via syringe. After stirring at 23 °C for 24 h, the reaction mixture was diluted with *d₃*-acetonitrile (3.0 mL).

The yield of **2g** was determined via ¹H NMR spectroscopy using ethyl acetate (5.0 µL, 51. µmol) as an internal standard. Comparison of the integral of the peak of **2g** at 7.46 ppm (aromatic C-H, 1 H) with that of the peak of ethyl acetate at 1.20 ppm (CH₃, 3H) revealed a 94% yield of **2g**.

### 1-(Chloromethyl)-4-(4-chlorophenyl)-1A-diazabicyclo[2.2.2]Octane²⁺ (2b) from 1,4-dichlorobenzene

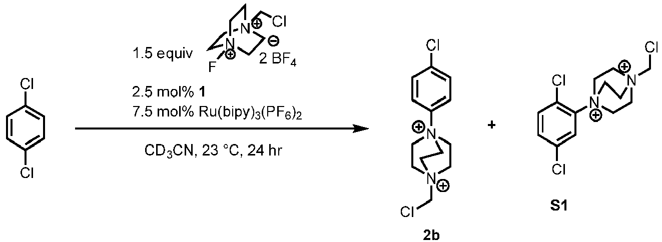

Palladium complex **1** (11.3 mg, 17.8 µmol) and Ru(bipy)₃(PF₆)₂ (45.9 mg, 53.4 µmol) were dissolved in *d₃*-acetonitrile (3.56 mL) to afford a stock solution. A 4 mL vial was charged with Selectfluor (47.3 mg, 0.134 mmol, 1.50 equiv) and 1,4-dichlorobenzene (13.1 mg, 89.0 µmol, 1.00 equiv). The stock solution (445 µL, c = 0.200 M) containing 1 (2.23 µmol, 2.50 mol%) and Ru(bipy)₃(PF₆)₂ (6.68 µmol, 7.50 mol%) was added via syringe. After stirring at 23 °C for 24 h, the reaction mixture was diluted with *d₃*-acetonitrile (2.0 mL). The yield of **2b** was determined via ¹H NMR spectroscopy using ethyl acetate (5.0 µL, 51. µmol) as an internal standard. Comparison of the integral of the peak of **2b** at 7.74-7.72 ppm (aromatic C-H, 2H) with that of the peak of ethyl acetate at 1.20 ppm (CH₃, 3H) revealed an 48% yield of **2b.**

Another compound, consistent with structure **S1,** was observed in 11% yield:

### 1-(Chloromethyl)-4-(4-methoxyphenyl)-1,4-diazabicyclo[2.2.2]octane²⁺ (2d) from 4-chloroanisole

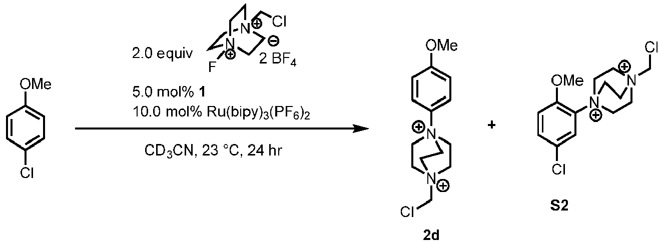

A stock solution was prepared, containing 1 (11.4 mg, 15.0 µmol) and Ru(bipy)₃(PF₆)₂ (25.8 mg, 30.0 µmol) were dissolved in *d₃*-acetonitrile (1.50 mL) to afford a stock solution.

A 4 mL vial was charged with Selectfluor (35.4 mg, 0.100 mmol, 2.0 equiv) and 4-chloroanisole (6.1 µL, 0.050 mmol, 1.0 equiv). The stock solution (250 µL, c = 0.20 M) containing **1** (2.5 µmol, 5.0 mol%) and Ru(bipy)₃(PF₆)₂ (5.00 µmol, 10.0 mol%) was added via syringe. After stirring at 23 °C for 48 h, the reaction mixture was diluted with *d*₃-acetonitrile (0.50 mL).
The yield of **2d** was determined via ¹H NMR spectroscopy using ethyl acetate (5.0 µL, 51. µmol) as an internal standard. Comparison of the integral of the peak of **2d** at 7.21-7.17 ppm (aromatic C-H, 2H) with that of the peak of ethyl acetate at 1.20 ppm (CH₃, 3H) revealed an 26% yield of **2d.**
The above reaction was repeated with 5 equivalents of 4-chloroanisole in order to isolate the Ar-TEDA products from other products.

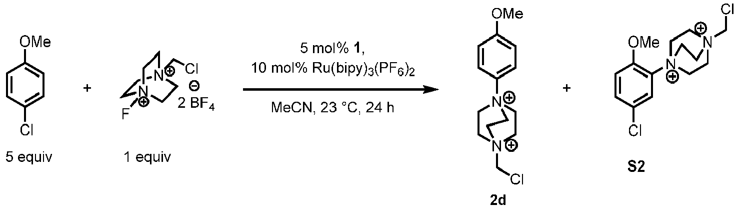

To a 20 mL vial were weighed **1** (31.8 mg, 50.0 µmol, 5.00 mol%), Ru(bipy)₃(PF₆)₂ (86.0 mg, 0.100 mmol, 10.0 mol%), and Selectfluor (354. mg, 1.00 mmol, 1.00 equiv). Acetonitrile was added (5.0 mL, c = 0.20 M), followed by 4-chloroanisole (612. µL, 5.00 mmol, 5.00 equiv). The mixture was stirred for 24 hours at room temperature, after which the mixture was diluted with 10 mL acetonitrile, then filtered through celite. The filtrate was concentrated, and the residue was triturated with 20 mL of 9:1 dichloromethane:methanol. The solid was collected by filtration, then washed with 10 mL 9:1 dichloromethane:methanol, then dichloromethane (3 × 10 mL). The solid was dried in vacuo to yield 316. mg of a tan solid. ¹H NMR analysis revealed 2c as the dominant Ar-TEDA product, along with ca. 29% of **S2:**

### (S)-1-(Chloromethyl)-4-(4-(2-(1,3-dioxoisoindolin-2-yl)-3-methoxy-3-oxopropyl)phenyl)-1,4-diazabicyclo[2.2.2]octane-1,4-diium (2u)

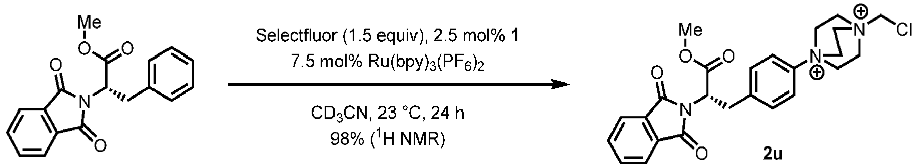

Palladium complex **1** (3.5 mg, 5.5 µmol) and Ru(bipy)₃(PF₆)₂ (14.2 mg, 16.5 µmol) were dissolved in *d*₃-acetonitrile (1.10 mL) to afford a stock solution. A 4 mL vial was charged with Selectfluor (53.1 mg, 0.150 mmol, 1.50 equiv) and methyl (S)-2-(1,3-dioxoisoindolin-2-yl)-3-phenylpropanoate (30.9 mg, 0.100 mmol, 1.00 equiv). The stock solution (1.0 mL, c = 0.20 M) containing **1** (2.5 µmol, 2.5 mol%) and Ru(bipy)₃(PF₆)₂ (7.50 µmol, 7.50 mol%) was added via syringe. After stirring at 23 °C for 24 h, the reaction mixture was diluted with *d*₃-acetonitrile (2.0 mL). The yield of **2u** was determined via ¹H NMR spectroscopy using *N,N-*dimethylformamide (5.0 µL, 64. µmol) as an internal standard. Comparison of the integral of the peak of **2u** at 4.28-4.30 ppm (alkyl C-H, 6H) with that of the peak of *N,N-*dimethylformamide at 2.89 ppm (CH₃, 3H) revealed an 98% yield of **2u.**

### 1-(5-(4-Chlorobenzoyl)-2-((1-isopropoxy-2-methyl-1-oxopropan-2-yl)oxy)phenyl)-4-(chloromethyl)-1,4-diazabicyclo[2.2.2]octane-1,4-diium (2x)

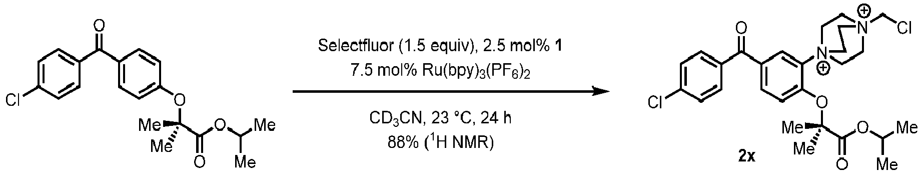

Palladium complex **1** (3.5 mg, 5.5 µmol) and Ru(bipy)₃(PF₆)₂ (14.2 mg, 16.5 µmol) were dissolved in *d*₃-acetonitrile (1.10 mL) to afford a stock solution. A 4 mL vial was charged with Selectfluor (53.1 mg, 0.150 mmol, 1.50 equiv) and fenofibrate (36.1 mg, 0.100 mmol, 1.00 equiv). The stock solution (1.0 mL, c = 0.20 M) containing **1** (2.5 µmol, 2.5 mol%) and Ru(bipy)₃(PF₆)₂ (7.50 µmol, 7.50 mol%) was added via syringe. After stirring at 23 °C for 24 h, the reaction mixture was diluted with *d*₃-acetonitrile (2.0 mL). The yield of **2x** was determined via ¹H NMR spectroscopy using *N,N-*dimethylformamide (5.0 µL, 64. µmol) as an internal standard. Comparison of the integral of the peak of **2x** at 4.61-4.64 ppm (alkyl C-H, 6H) with that of the peak of *N,N-*dimethylformamide at 2.89 ppm (CH₃, 3H) revealed an 88% yield of **2x**.

### 1-(Chloromethyl)-4-(3-((3-chlorophenethyl)carbamoyl)-4-methylphenyl)-1,4-diazabicyclo[2.2.2]octane-1,4-diium (2h)

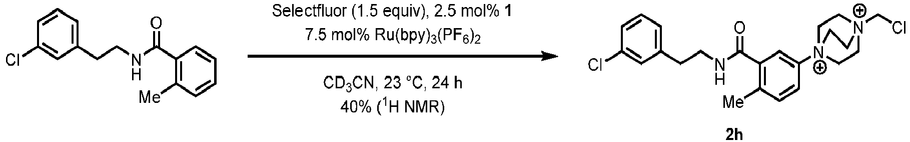

Palladium complex **1** (7.2 mg, 11. µmol) and Ru(bipy)₃(PF₆)₂ (29.0 mg, 33.8 µmol) were dissolved in *d₃*-acetonitrile (2.25 mL) to afford a stock solution. A 4 mL vial was charged with Selectfluor (26.6 mg, 75.0 µmol, 1.50 equiv) and *N-*(3-chlorophenethyl)-2-methylbenzamide (13.7 mg, 50.0 µmol, 1.00 equiv). The stock solution (0.25 mL, c = 0.20 M) containing **1** (1.2 µmol, 2.5 mol%) and Ru(bipy)₃(PF₆)₂ (3.75 µmol, 7.50 mol%) was added via syringe. After stirring at 23 °C for 24 h, the reaction mixture was diluted with *d*₃-acetonitrile (2.0 mL). The yield of **2h** was determined via ¹H NMR spectroscopy using *N,N-*dimethylformamide (2.0 µL, 26. µmol) as an internal standard. Comparison of the integral of the peak of 2h at 7.66 ppm (aromatic C-H, 1 H) with that of the peak of *N,N-*dimethylformamide at 2.89 ppm (CH, 1 H) revealed an 40% yield of **2h.**

### Procedure for aromatic C-H TEDAylation reactions (excess arene)

### 1-(Chloromethyl)-4-(4-fluorophenyl)-1,4-diazabicyclo[2.2.2]octane²⁺ (2a)

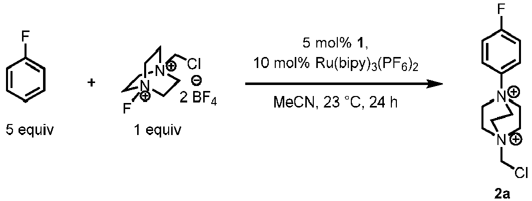

To a 20 mL vial were added palladium complex **1** (31.8 mg, 50.0 µmol, 5.00 mol%), Ru(bipy)₃(PF₆)₂ (86.0 mg, 100. µmol, 10.0 mol%), Selectfluor (354. mg, 1.00 mmol, 1.00 equiv), and fluorobenzene (0.530 mL, 5.00 mmol, 5.00 equiv), and acetonitrile (2.5 mL, c = 0.20 M). The reaction mixture was stirred at 40 °C for 48 h. The reaction mixture was diluted with acetonitrile, filtered through celite, and the filtrate was concentrated *in vacuo.* The residue was triturated with 20 mL methanol:dichloromethane. The solid was collected by filtration on a glass frit, washed with 10 mL 1:9 methanol:dichloromethane, followed by 3 × 10 mL dichloromethane, then dried *in vacuo* to afford 341. mg of a tan powder. For yield determination, an NMR sample in *d*₃-acetonitrile was prepared containing 10.0 mg of the product mixture and 5.0 µL of ethyl acetate (51. µmol) as internal standard. Comparison of the integral of the peak of **2a** at 4.45-4.37 ppm (3 × CH₂, 6H) with that of the peak of ethyl acetate at 1.20 ppm (CH₃, 3H) revealed the bulk mixture to contain 0.60 mmol of Ar-TEDA (60% yield). Also present was 0.18 mmol H-TEDA²⁺ (18%), measured by integration of the peak at 3.85-3.81 ppm (3 × CH₂, 6H).
NMR Spectroscopy: ¹H NMR (500 MHz, CD₃CN, 23 °C, δ): 7.91-7.84 (m, 2H), 7.49-7.42 (m, 2H), 5.36 (s, 2H), 4.45-4.37 (m, 6H), 4.18-4.11 (m, 6H). ¹³C NMR (125 MHz, CD₃CN, 23 °C, δ): 163.4 (d, *J* = 252.4 Hz), 140.7 (s), 123.8 (d, *J* = 9.6 Hz), 118.9 (d, *J* = 24.0 Hz), 70.1 (s), 55.6 (s), 51.6 (s). ¹⁹F NMR (470 MHz, CD₃CN, 23 °C, δ): -110.0.
Mass spectrometry: HRMS-FIA(m/z) calcd for C₁₃H₁₇ClFN₂ [M-H]⁺, 255.1059; found, 255.1061.

### 1-(Chloromethyl)-4-(4-chlorophenyl)-1,4-diazabicyclo[2.2.2]octane²⁺ (2b)

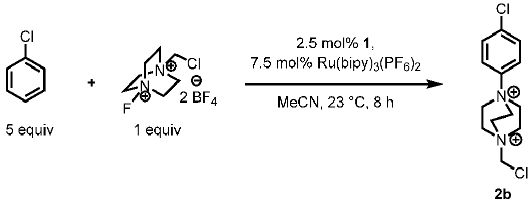

A 50 mL round-bottom flask was charged with palladium complex **1** (31.8 mg, 50.0 µmol. 5.00 mol%), Ru(bipy)₃(PF₆)₂ (86.0 mg, 0.100 mmol, 10.0 mol%), and Selectfluor (354. mg, 1.00 mmol, 1.00 equiv). Acetonitrile (5.0 mL, c = 0.20 M) was added, followed by chlorobenzene (509 µL, 5.00 mmol, 5.00 equiv) via syringe. After stirring at 23 °C for 19 h, the reaction mixture was filtered through celite and concentrated *in vacuo.* The residue was triturated with a solvent mixture of dichloromethane/methanol (9/1 (v/v), 10 mL) and dichloromethane (5 × 10 mL) at 23 °C to afford 463 mg of the title compound as a light yellow solid. For yield determination, an NMR sample in *d*3-acetonitrile was prepared containing 20.0 mg of the product mixture and 3.0 µL of ethyl acetate (31. µmol) as internal standard. Comparison of the integral of the peak of **2b** at 7.79-7.77 ppm (aromatic C-H, 2H) with that of the peak of ethyl acetate at 1.20 ppm (CH₃, 3H) revealed the bulk mixture to contain 0.80 mmol of Ar-TEDA (80% yield). Also present was 0.14 mmol H-TEDA²⁺ (14%), measured by integration of the peak at 3.85-3.81 ppm (3 × CH₂, 6H).
NMR Spectroscopy: ¹H NMR (600 MHz, CD₃CN, 23 °C, δ): 7.79-7.82 (m, 2H), 7.70-7.73 (m, 2H), 5.36 (s, 2H), 4.40-4.43 (m, 6H), 4.13-4.15 (m, 6H). ¹³C NMR (125 MHz, CD₃CN, 23 °C, δ): 142.9, 137.9, 131.6, 122.9, 69.7, 55.1, 51.2.
Mass Spectrometry: HRMS-FIA(m/z) calcd for C₁₃H₁₈Cl₂N₂²⁺ [M]²⁺, 136.0418; found, 136.0419.

### 1-(Chloromethyl)-4-(4-methyl-phenyl)-1,4-diazabicyclo[2.2.2]octane²⁺ (2c)

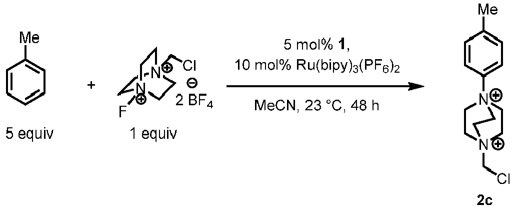

To a 20 mL vial were added palladium complex **1** (31.8 mg, 50.0 µmol, 5.00 mol%), Ru(bipy)₃(PF₆)₂ (86.0 mg, 100. µmol, 10.0 mol%), Selectfluor (354. mg, 1.00 mmol, 1.0 equiv), and toluene (0.530 mL, 5.00 mmol, 5.00 equiv), and acetonitrile (2.5 mL, c = 0.20 M). The reaction mixture was stirred at 23 °C for 48 h. The reaction mixture was concentrated *in vacuo,* then triturated with CH₂Cl₂ to afford 324 mg of a tan powder. For yield determindation, an NMR sample in *d*₃-acetonitrile was prepared containing 10.0 mg of the product mixture and 5.0 µL of ethyl acetate (51. µmol) as internal standard. Comparison of the integral of the peak of **2c** at 4.37-4.32 ppm (3 × CH₂, 6H) with that of the peak of ethyl acetate at 1.20 ppm (CH₃, 3H) revealed the bulk mixture to contain 0.59 mmol of Ar-TEDA (59% yield). Also present was 0.18 mmol H-TEDA²⁺ (18%), measured by integration of the peak at 3.85-3.81 ppm (3 × CH₂, 6H).
NMR Spectroscopy: ¹H NMR (600 MHz, CD₃CN, 23 °C, δ): 7.67-7.63 (m, 2H), 7.54-7.00 (m, 2H), 5.33 (s, 2H), 4.37-4.32 (m, 6H), 4.11-4.07 (m, 6H), 2.44 (s, 3H). ¹³C NMR (125 MHz, DMSO, 23 °C, δ): 142.1, 141.4, 131.0, 120.3, 68.2, 53.94, 50.4, 20.3.
Mass spectrometry: HRMS-FIA(m/z) calcd for C₁₄H₂₀ClN₂⁺ [M-H]⁺, 251.1310; found, 251.1312.

### 1-(Chloromethyl)-4-(4-methyl-phenyl)-1,4-diazabicyclo[2.2.2]octane²⁺ (2d)

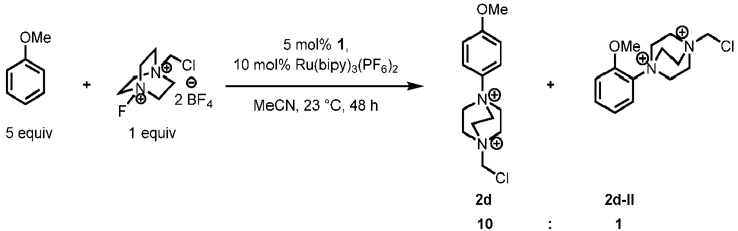

To a 20 mL vial were added palladium complex 1 (31.8 mg, 50.0 µmol, 5.00 mol%), Ru(bipy)₃(PF₆)₂ (86.0 mg, 100. µmol, 10.0 mol%), Selectfluor (354. mg, 1.00 mmol, 1.0 equiv), and anisole (0.544 mL, 5.00 mmol, 5.0 equiv), and acetonitrile (2.5 mL, c = 0.20 M). The reaction mixture was stirred at 23 °C for 48 h. The reaction mixture was concentrated *in vacuo,* then triturated with CH₂Cl₂ to afford 324. mg of a tan powder. For yield determination, an NMR sample in *d₃*-acetonitrile was prepared containing 10.0 mg of the product mixture and 5.0 µL of ethyl acetate (51. µmol) as internal standard. Comparison of the integral of the peak of **2d** at 4.36-4.31 ppm (3 × CH₂, 6H) with that of the peak of ethyl acetate at 1.20 ppm (CH₃, 3H) revealed the bulk mixture to contain 0.43 mmol of **2d** (43% yield). An additional compound was observed in 4.3% yield, assignable to **2d-II;** (upon reduction, *N*-(2-methoxyphenyl)piperazine **(3e-II)** is observed in small amounts,). Also present was 0.31 mmol H-TEDA²⁺ (31%), measured by integration of the peak at 3.85-3.81 ppm (3 × CH₂, 6H).
NMR Spectroscopy: ¹H NMR (600 MHz, CD₃CN, 23 °C, δ): 7.72-7.67 (m, 2H), 7.21-7.17 (m, 2H), 5.33 (s, 2H), 4.36-4.31 (m, 6H), 4.20-4.15 (m, 6H), 3.88 (s, 3H). ¹³C NMR (125 MHz, DMSO, 23 °C, δ): 160.5, 137.0, 122.1, 115.5, 68.2, 54.1, 50.4, 43.4.

Mass spectrometry: HRMS-FIA(m/z) calcd for C₁₄H₂₀ClN₂O⁺ [M-H]⁺, 267.1259; found, 267.1263.

### 1-(Chloromethyl)-4-(3-fluoro-4-methoxyphenyl)-1,4-diazabicyclo[2.2.2]octane²⁺ (2e)

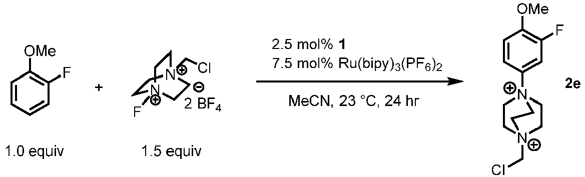

A 50 mL round-bottom flask was charged with palladium complex 1 (31.8 mg, 50.0 µmol. 5.00 mol%), Ru(bipy)₃(PF₆)₂ (86.0 mg, 0.100 mmol, 10.0 mol%), and Selectfluor (531. mg, 1.50 mmol, 1.50 equiv). Acetonitrile (5.0 mL, c = 0.20 M) was added, followed by 2-fluoroanisole (112 µL, 1.00 mmol, 1.00 equiv) via syringe. After stirring at 23 °C for 24 h, the reaction mixture was filtered through celite and concentrated *in vacuo.* The residue was triturated with a solvent mixture of dichloromethane/methanol (9/1 (v/v), 10 mL) and dichloromethane (5 × 10 mL) at 23 °C to afford 573 mg of a orange solid. The solid was triturated with a solvent mixture of dichloromethane/methanol (9/1 (v/v), 10 mL) and dichloromethane (5 × 10 mL) at 23 °C to afford 562 mg of the title compound as a light orange solid. For yield determination, an NMR sample in *d₃*-acetonitrile was prepared containing 20.0 mg of the product mixture and 3.0 µL of ethyl acetate (3.1 × 10⁻⁵ mol) as internal standard. Comparison of the integral of the peak of **2e** at 7.34 ppm (aromatic C-H, 1 H) with that of the peak of ethyl acetate at 1.20 ppm (CH₃, 3H) revealed the bulk mixture to contain 0.76 mmol of Ar-TEDA (76% yield). Also present was 0.42 mmol H-TEDA²⁺ (42%) measured by integration of the peak at 3.85-3.81 ppm (3 × CH₂, 6H). NMR Spectroscopy: ¹H NMR (600 MHz, CD₃CN, 23 °C, δ): 7.67 (dd, *J* = 12.2, 3.3 Hz, 1 H), 7.59 (ddd, *J =* 9.3, 3.3, 1.6 Hz, 1 H), 7.34 (dd, *J =* 9.3, 9.3 Hz, 1 H), 5.36 (s, 2H), 4.36-4.38 (m, 6H), 4.11-4.13 (m, 6H), 3.96 (s, 3H). ¹³C NMR (125 MHz, CD₃CN, 23 °C, δ): 152.0 (d, *J* = 249.4 Hz), 150.3 (d, *J* = 9.7 Hz), 135.9 (d, *J* = 7.8 Hz), 117.5 (d, *J* = 3.8 Hz), 114.9 (d, *J* = 2.8 Hz), 110.0 (d, *J* = 25.0 Hz), 69.5, 55.1, 51.0, 50.4. ¹⁹F NMR (475 MHz, CD₃CN, 23 °C, δ): -130.2.
Mass Spectrometry: HRMS-FIA(m/z) calcd for C₁₄H₂₀ClFN₂O²⁺ [M]²⁺, 143.0619; found, 143.0626.

### 1-(Chloromethyl)-4-(fluorenon-2-yl)-1,4-diazabicyclo[2.2.2]octane²⁺ (2f)

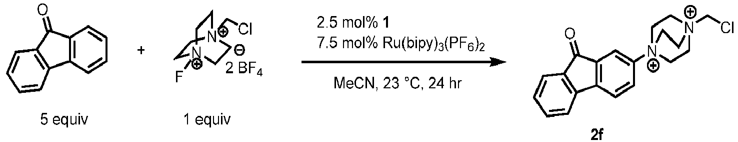

A 50 mL round-bottom flask was charged with palladium complex **1** (31.8 mg, 50.0 µmol. 5.00 mol%), Ru(bipy)₃(PF₆)₂ (86.0 mg, 0.100 mmol, 10.0 mol%), Selectfluor (354. mg, 1.00 mmol, 1.00 equiv), and 9*H*-fluoren-9-one (901 mg, 5.00 mmol, 5.00 equiv). Acetonitrile (5.0 mL, c = 0.20 M) was added via syringe. After stirring at 23 °C for 16 h, the reaction mixture was filtered through celite and concentrated *in vacuo.* The residue was triturated with a solvent mixture of dichloromethane/methanol (9/1 (v/v), 10 mL) and dichloromethane (5 × 10 mL) at 23 °C to afford 559. mg of a yellow solid. The solid was triturated with a solvent mixture of dichloromethane/methanol (9/1 (v/v), 10 mL) and dichloromethane (5 × 10 mL) at 23 °C to afford 468. mg of a bright yellow solid. The solid was triturated with a solvent mixture of dichloromethane/methanol (9/1 (v/v), 10 mL) and dichloromethane (5 × 10 mL) at 23 °C to afford 453. mg of the title compound as a bright yellow solid. For yield determination, an NMR sample in *d₃*-acetonitrile was prepared containing 20.0 mg of the product mixture and 3.0 µL of ethyl acetate (3.1 × 10⁻⁵ mol) as internal standard. Comparison of the integral of the peak of **2f** at 5.36 ppm (CH₂Cl, 2H) with that of the peak of ethyl acetate at 1.20 ppm (CH₃, 3H) revealed the bulk mixture to contain 0.77 mmol of Ar-TEDA (77% yield). Also present was 0.069 mmol H-TEDA²⁺ (7%), measured by integration of the peak at 3.85-3.81 ppm (3 × CH₂, 6H).
The product mixture of **2f** obtained above (50 mg) was further purified through repeated recrystallization by vapor diffusion of diethyl ether into an acetonitrile solution. Pure product **2f** (15 mg) was obtained as yellow crystals and characterized.
Note: Compound **2f** exhibits concentration-dependent chemical shifts. The chemical shifts listed below were recorded from a sample of 12 mg **2f** in 7.5 mL CD₃CN.
NMR Spectroscopy: ¹H NMR (600 MHz, CD₃CN, 23 °C, δ): 8.00-7.97 (m, 2H), 7.95-7.92 (m, 1H), 7.80-7.77 (m, 1H), 7.71-7.65 (m, 2H), 7.51-7.47 (m, 1H), 5.38 (s, 2H), 4.45-4.47 (m, 6H), 4.15-4.18 (m, 6H). ¹³C NMR (125 MHz, CD₃CN, 23 °C, δ): 191.7, 147.7, 145.0, 143.0, 136.8, 136.7, 135.0, 131.7, 127.7, 125.4, 123.8, 123.1, 116.9, 70.0, 55.4, 51.5.
Mass Spectrometry: HRMS-FIA(m/z) calcd for C₂₀H₂₁ClN₂O²⁺ [M]²⁺, 170.0666; found, 170.0672.

### 1-(Chloromethyl)-4-(5-methoxy-2-(methoxycarbonyl)phenyl)²⁺(2g)

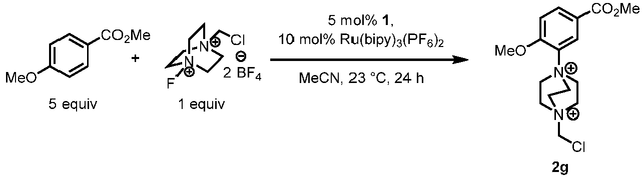

To a 20 mL vial were added palladium complex **1** (19.0 mg, 25.0 µmol, 5.00 mol%), Ru(bipy)₃(PF₆)₂ (43.0 mg, 5.00 µmol, 10.0 mol%), Selectfluor (177. mg, 0.500 mmol, 1.00 equiv), and methyl 4-methoxybenzoate (416 mg, 2.50 mmol, 5.00 equiv), and acetonitrile (2.5 mL, c = 0.20 M). The reaction mixture was stirred at 23 °C for 48 h. The reaction mixture was concentrated *in vacuo,* then triturated with CH₂Cl₂ to afford 191. mg of a tan powder. ¹H NMR shows ca. 35% contamination by H-TEDA²⁺.
NMR Spectroscopy: ¹H NMR (500 MHz, CD₃CN, 23 °C, δ): 8.27 (dd, *J =* 8.9, 1.8 Hz, 1 H), 8.21 (d, *J =* 1.8 Hz, 1 H), 7.46 (d, *J =* 8.9 Hz, 1 H), 5.33 (s, 2H), 4.62-4.54 (m, 6H), 4.15-4.09 (m, 6H), 4.14 (s, 3H), 3.92 (s, 3H). ¹³C NMR (125 MHz, CD₃CN, 23 °C, δ): 165., 156.51, 135.4, 130.8, 124.7, 124.3, 116.6, 70.4, 58.4, 53.7, 51.6, 45.1.
Mass spectrometry: HRMS-FIA(m/z) calcd for C₁₆H₂₂ClN₂O₃⁺ [M-H]⁺, 325.1313; found, 325.1315.

### 1-(Chloromethyl)-4-(3-((3-chlorophenethyl)carbamoyl)-4-methylphenyl)-1,4-diazabicyclo[2.2.2]octane-1,4-diium (2h)

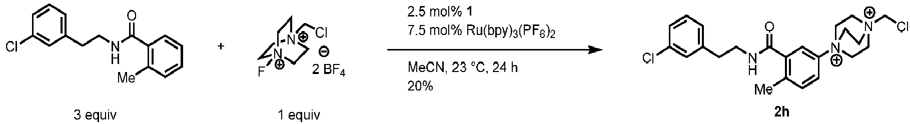

A 4 mL vial was charged with palladium complex **1** (3.2 mg, 5.0 µmol.2.5 mol%), Ru(bpy)₃(PF₆)₂ (12.9 mg, 15.0 µmol, 7.50 mol%), Selectfluor (70.9 mg, 0.200 mmol, 1.00 equiv), and *N-*(3-chlorophenethyl)-2-methylbenzamide (164. mg, 0.600 mmol, 3.00 equiv). Acetonitrile (1.0 mL, c = 0.20 M) was added via syringe, and the reaction mixture was stirred at 23 °C for 24 h. The reaction mixture was concentrated *in vacuo* to afford a red heterogeneous solid mixture. The solid was triturated with dichloromethane to afford 62 mg of the title compound as a light yellow solid (20% yield). Also present was ca. 49% contamination by H-TEDA²⁺, measured by integration of the peak at 3.85-3.81 ppm (3 × CH₂, 6H).
NMR Spectroscopy: ¹H NMR (600 MHz, CD₃CN, 23 °C, δ): 7.71 (dd, *J* = 8.8, 2.9 Hz, 1 H), 7.67 (d, *J =* 2.9 Hz, 1 H), 7.53 (d, *J =* 8.8 Hz, 1 H), 7.30-7.33 (m, 2H), 7.23-7.25 (m, 2H), 6.99 (bs, 1 H), 5.36 (s, 2H), 4.38-4.40 (m, 6H), 4.12-4.15 (m, 6H), 3.61-3.64 (m, 2H), 2.91 (t, *J* = 6.9 Hz, 2H), 2.34 (s, 3H). ¹³C NMR (125 MHz, CD₃CN, 23 °C, δ): 168.2, 142.9, 142.0, 141.3, 140.1, 134.6, 134.1, 131.0, 129.9, 128.6, 127.3, 121.9, 120.0, 70.1, 55.4, 51.6, 41.4, 35.8, 19.3.
Mass Spectrometry: HRMS-FIA(m/z) calcd for C₂₃H₂₉Cl₂N₃O²⁺ [M]2+, 216.5838; found, 216.5846.

### 1-(Chloromethyl)-4-phenyl-1,4-diazabicyclo[2.2.2]octane²⁺ (2i)

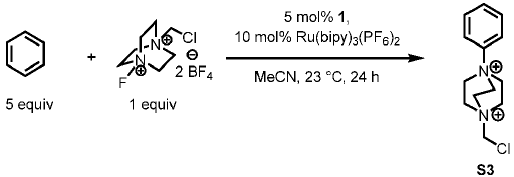

To a 20 mL vial were added palladium complex **1** (31.8 mg, 50.0 µmol, 5.00 mol%), Ru(bipy)₃(PF₆)₂ (86.0 mg, 100. µmol, 10.0 mol%), Selectfluor (354. mg, 1.00 mmol, 1.00 equiv), and benzene (0.446 mL, 5.00 mmol, 5.0 equiv), and acetonitrile (2.5 mL, c = 0.20 M). The reaction mixture was stirred at 23 °C for 48 h. The reaction mixture was concentrated *in vacuo,* then triturated with CH₂Cl₂ to afford 360 mg of a tan powder. For yield determination, an NMR sample in *d₃*-acetonitrile was prepared containing 10.0 mg of the product mixture and 5.0 µL of ethyl acetate (51. µmol) as internal standard. Comparison of the integral of the peak of **S1** at 4.41-4.36 ppm (3 × CH₂, 6H) with that of the peak of ethyl acetate at 1.20 ppm (CH₃, 3H) revealed the bulk mixture to contain 0.67 mmol of Ar-TEDA (67% yield). Also present was 0.15 mmol H-TEDA²⁺ (15%), measured by integration of the peak at 3.85-3.81 ppm (3 × CH₂, 6H).
NMR Spectroscopy: ¹H NMR (600 MHz, CD₃CN, 23 °C, δ): 7.82-7.77 (m, 2H); 7.75-7.69 (m, 3H), 5.35 (s, 2H), 4.41-4.36 (m, 6H), 4.41-4.09 (m, 6H). ¹³C NMR (125 MHz, CD₃CN, 23 °C, δ): 144.4, 131.3, 130.8, 120.7, 68.2, 53.9, 50.4
Mass spectrometry: HRMS-FIA(m/z) calcd for C₁₃H₁₈ClN₂⁺ [M-H]⁺, 237.1153; found, 237.1154.

### Procedure for aromatic C-H TEDAylation reactions (excess arene)

### 1-methyl-4-(4-methoxyphenyl)-1,4-diazabicyclo[2.2.2]octane²⁺ bis-anion (2j)

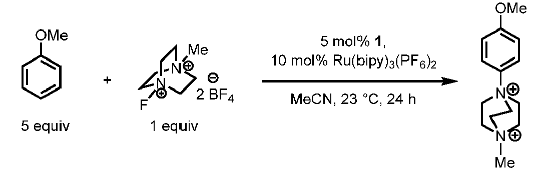

To a 20 mL vial were added palladium complex **1** (15.9 mg, 25.0 µmol, 5.00 mol%), Ru(bipy)₃(PF₆)₂ (43.0 mg, 50.0 µmol, 10.0 mol%), Selectfluor-II (159.9 mg, 0.5000 mmol, 1.00 equiv), and anisole (0.272 mL, 2.50 mmol, 5.00 equiv), and acetonitrile (2.5 mL, c = 0.10 M). The reaction mixture was stirred at 23 °C for 48 h. The reaction mixture was diluted with acetonitrile (2.5 mL), filtered through celite, and the filtrate was concentrated *in vacuo.* The residue was triturated with 10 mL methanol:dichloromethane (1:9). The solid was collected by filtration on a glass frit, washed twice with 10 mL methanol:dichloromethane (1:9), then dried *in vacuo* to afford 136. mg of a tan powder (0.333 mmol, 66%).
NMR Spectroscopy: ¹H NMR (500 MHz, CD₃CN, 23 °C, δ): 7.70-7.65 (m, 2H), 7.19-7.15 (m, 2H), 5.36 (s, 2H), 4.30-4.26 (m, 6H), 4.02-3.97 (m, 6H), 3.89 (s, 3H), 3.36 (s, 3H).

### Representative C-H piperazination procedure:

### 1-(p-Tolyl)piperazine (3b)

A 100 mL pressure tube was charged with palladium complex **1** (13.6 mg, 21.4 µmol.2.50 mol%), Ru(bipy)₃(PF₆)₂ (55.2 mg, 64.2 µmol, 7.50 mol%), and Selectfluor (455. mg, 1.28 mmol, 1.50 equiv). Acetonitrile (4.3 mL, c = 0.20 M) was added, followed by toluene (91.1 µL, 0.856 mmol, 1.00 equiv) via syringe. The reaction mixture was stirred at 23 °C for 24 h. Saturated aqueous sodium thiosulfate (8.6 mL) and water (8.6 mL) were added, the pressure tube was sealed, and the reaction mixture was stirred at 100 °C for 2 h. After cooling to 23 °C, the reaction mixture was transferred to a separatory funnel. Dichloromethane (20 mL) and ethylenediamine (1.5 mL) were added and the organic layer was washed with 6 M aqueous sodium hydroxide (5 mL). The aqueous layer was extracted with dichloromethane (2 × 10 mL). The combined organic layers were extracted with 1 M aqueous hydrochloric acid (2 × 15 mL). Ethylenediamine (5.0 mL) was added to the combined acidic aqueous layers, followed by basification with 6 M aqueous sodium hydroxide (8 mL). The basic aqueous layer was extracted with dichloromethane (3 × 15 mL). The combined organic layers were dried over sodium sulfate, filtered, and concentrated *in vacuo* to afford a red oil. The residue was purified by chromatography on silica gel eluting with a solvent mixture of dichloromethane/methanol/28% aqueous ammonium hydroxide (97.5/2.0/0.5 (v/v/v)) to afford 119. mg of the title compound as a yellow oil (79% yield).

### Procedures for the preparation of aryl piperazines

### 1-(3,4-Dimethylphenyl)piperazine (3f)

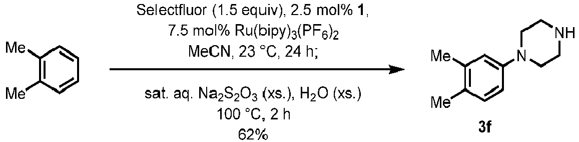

A 100 mL pressure tube was charged with palladium complex **1** (13.2 mg, 20.7 µmol.2.50 mol%), Ru(bipy)₃(PF₆)₂ (53.4 mg, 62.1 µmol, 7.50 mol%), and Selectfluor (440. mg, 1.24 mmol, 1.50 equiv). Acetonitrile (5.0 mL, c = 0.20 M) was added, followed by *o*-xylene (100. µL, 1.00 mmol, 1.00 equiv) via syringe. The reaction mixture was stirred at 23 °C for 24 h. Saturated aqueous sodium thiosulfate (8.3 mL) and water (8.3 mL) were added, the pressure tube was sealed, and the reaction mixture was stirred at 100 °C for 2 h. After cooling to 23 °C, ethylenediamine (5.0 mL) and water (15 mL) were added, and the mixture was stirred for 1 h further. The reaction mixture was transferred to a separatory funnel. Dichloromethane (20 mL) was added and the organic layer was separated. The aqueous layer was extracted with dichloromethane (2 × 15 mL). The combined organic layers were extracted with 1 M aqueous hydrochloric acid (3 × 10 mL). Ethylenediamine (2.0 mL) was added to the combined acidic aqueous layers, followed by basification with 6 M aqueous sodium hydroxide (6 mL). The basic aqueous layer was extracted with dichloromethane (3 × 10 mL). The combined organic layers were dried over sodium sulfate, filtered, and concentrated *in vacuo* to afford a red solid (117. mg). The residue was purified by chromatography on silica gel eluting with a solvent mixture of dichloromethane/methanol/28% aqueous ammonium hydroxide (97.5/2.0/0.5 (v/v/v)) to afford 97.0 mg of the title compound as a yellow solid (62% yield).
R*_{f}* = 0.32 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 7.03 (d, *J* = 8.2 Hz, 1 H), 6.76 (d, *J* = 2.6 Hz, 1 H), 6.69 (dd, *J* = 8.2, 2.6 Hz, 1 H), 3.09 (m, 4H), 3.03 (m, 4H), 2.24 (s, 3H), 2.19 (s, 3H), 1.72 (s, 1H). ¹³C NMR (125 MHz, CDCl₃, 23 °C, δ): 150.3, 137.2, 130.2, 128.2, 118.2, 114.0, 51.2, 46.4, 20.3, 18.9.
Mass Spectrometry: HRMS-FIA(m/z) calcd for C₁₂H₁₉N₂ [M+H]⁺, 191.1543; found, 191.1538.

### 1-(3-(tert-Butyl)-4-methylphenyl)piperazine (3g), 1-(4-(tert-Butyl)-3-methylphenyl) piperazine (3g-II)

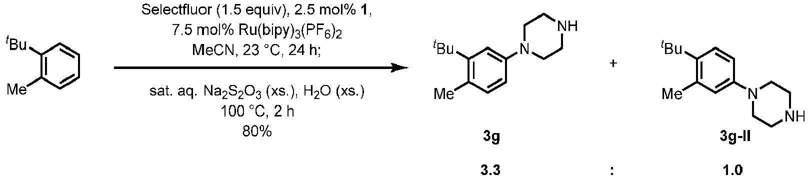

A 100 mL pressure tube was charged with palladium complex **1** (7.16 mg, 11.2 µmol.2.50 mol%), Ru(bipy)₃(PF₆)₂ (29.0 mg, 33.8 µmol, 7.50 mol%), and Selectfluor (239. mg, 0.675 mmol, 1.50 equiv). Acetonitrile (2.2 mL, c = 0.20 M) was added, followed by 1-(*tert-*butyl)-2-methylbenzene (75.0 µL, 0.450 mmol, 1.00 equiv) via syringe. The reaction mixture was stirred at 23 °C for 24 h. Saturated aqueous sodium thiosulfate (4.5 mL) and water (4.5 mL) were added, the pressure tube was sealed, and the reaction mixture was stirred at 100 °C for 2 h. After cooling to 23 °C, the reaction mixture was transferred to a separatory funnel. Dichloromethane (20 mL), ethylenediamine (0.5 mL), water (5 mL) and 6 M aqueous sodium hydroxide (0.5 mL) were added and the organic layer was separated. The aqueous layer was extracted with dichloromethane (2 × 15 mL). The combined organic layers were extracted with 1 M aqueous hydrochloric acid (3 × 10 mL). Ethylenediamine (2.0 mL) was added to the combined acidic aqueous layers, followed by basification with 6 M aqueous sodium hydroxide (5 mL). The basic aqueous layer was extracted with dichloromethane (3 × 15 mL). The combined organic layers were dried over sodium sulfate, filtered, and concentrated *in vacuo* to afford a red oil (91.4 mg). The residue was purified by chromatography on silica gel eluting with a solvent mixture of dichloromethane/methanol/28% aqueous ammonium hydroxide (96.5/3.0/0.5 (v/v/v)) to afford 83.5 mg of the title compounds **(3g:3g-II)** as a yellow oil (80% yield).
R*_{f}* = 0.25 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): **3g** 7.01-7.03 (m, 2H), 6.68 (dd, *J =* 8.3, 2.6 Hz, 1 H), 3.09-3.13 (m, 4H), 3.01-3.06 (m, 4H), 2.46 (s, 3H), 1.88 (s, 1 H), 1.40 (s, 9H). **3g-II** 7.26 (d, *J* = 8.7 Hz, 1 H), 6.67-6.72 (m, 2H), 3.09-3.13 (m, 4H), 3.01-3.06 (m, 4H), 2.51 (s, 3H), 1.38 (s, 9H). ¹³C NMR (125 MHz, CDCl₃, 23 °C, δ): 150.0, 149.6, 148.6, 139.5, 137.1, 133.4, 128.0, 126.9, 120.6, 115.6, 113.6, 113.0, 51.3, 50.4, 46.4, 46.3, 36.2, 35.2, 31.2, 30.8, 23.6, 22.5.
Mass Spectrometry: HRMS-FIA(m/z) calcd for C₁₅H₂₅N₂ [M+H]⁺, 233.2012; found, 233.2022.

### Methyl 2-methoxy-5-(piperazin-1-yl)benzoate (3h)

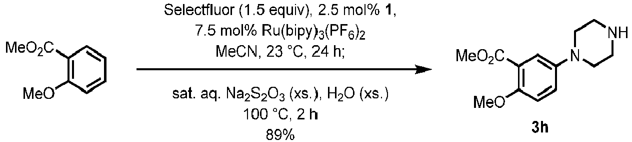

A 100 mL pressure tube was charged with palladium complex **1** (11.1 mg, 17.4 µmol.2.50 mol%), Ru(bipy)₃(PF₆)₂ (44.9 mg, 52.2 µmol, 7.50 mol%), and Selectfluor (370. mg, 1.04 mmol, 1.50 equiv). Acetonitrile (3.5 mL, c = 0.20 M) was added, followed by methyl 2-methoxybenzoate (100. µL, 0.700 mmol, 1.00 equiv) via syringe. The reaction mixture was stirred at 23 °C for 24 h. Saturated aqueous sodium thiosulfate (7.0 mL) and water (7.0 mL) were added, the pressure tube was sealed, and the reaction mixture was stirred at 100 °C for 2 h. After cooling to 23 °C, the reaction mixture was transferred to a separatory funnel. Dichloromethane (20 mL) and ethylenediamine (0.3 mL) were added and the organic layer was separated. The aqueous layer was extracted with dichloromethane (2 × 15 mL). The combined organic layers were extracted with 10% aqueous glacial acetic acid (2 × 15 mL). The combined acidic aqueous layers were basified with ethylenediamine (4.0 mL). The basic aqueous layer was extracted with dichloromethane (3 × 15 mL). The combined organic layers were dried over sodium sulfate, filtered, and concentrated *in vacuo* to afford a red oil (175. mg). The residue was purified by chromatography on silica gel eluting with a solvent mixture of dichloromethane/methanol/28% aqueous ammonium hydroxide (96.5/3.0/0.5 (v/v/v)) to afford 155. mg of the title compound as a yellow oil (89% yield).
R*_{f}* = 0.16 (dichloromethane/methanol/28% aqueous ammonium hydroxide 90.5:9.0:0.5 (v/v/v)). NMR Spectroscopy: ¹H NMR (125 MHz, CDCl₃, 23 °C, δ): 7.35 (d, *J* = 3.0 Hz, 1 H), 7.03 (dd, *J =* 9.0, 3.0 Hz, 1 H), 6.88 (d, *J =* 9.0 Hz, 1 H), 3.85 (s, 3H), 3.82 (s, 3H), 2.98-3.03 (m, 8H), 1.67 (s, 1H). ¹³C NMR (500 MHz, CDCl₃, 23 °C, δ): 166.9, 153.2, 145.6, 122.1, 120.3, 119.8, 113.5, 56.6, 52.1, 51.5, 46.2.
Mass Spectrometry: HRMS-FIA(m/z) calcd for C₁₃H₁₈N₂O₃Na [M+Na]⁺, 273.1210; found, 273.1207.

### 1-(3-Fluoro-4-methoxyphenyl)piperazine (3j)

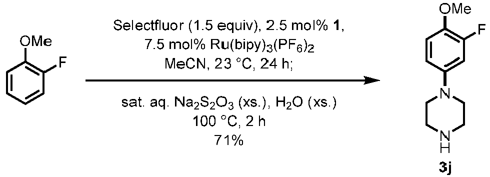

A 100 mL pressure tube was charged with palladium complex **1** (15.9 mg, 25.0 µmol.2.50 mol%), Ru(bipy)₃(PF₆)₂ (64.5 mg, 75.0 µmol, 7.50 mol%), Selectfluor (531.5 mg, 1.500 mmol, 1.50 equiv), and 2-fluoroanisole (112 µL, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 1 h. After cooling to 23 °C, the reaction mixture was filtered through celite, and the filter cake was extracted with 5 × 15 mL acetonitrile and 2 × 15 mL water. The acetonitrile was removed from the filtrate by rotary evaporation. To the remaining aqueous mixture 0.50 mL ethylene diamine was added, and the aqueous mixture was basified to pH 14 with 6 M NaOH, then transferred to a separatory funnel. The aqueous layer was extracted with dichloromethane (5 × 15 mL). The combined organic layers were extracted with 1 M HCl (5 × 15 mL). To the combined acidic aqueous layers was added ethylene diamine (0.5 mL), and the mixture was basified to pH 14 with 6 M NaOH. The basic aqueous layer was extracted with dichloromethane (5 × 15 mL). The combined organic layers were dried over sodium sulfate, filtered, and concentrated *in vacuo* to afford a red oil. The residue was purified by chromatography on silica gel eluting with dichloromethane/methanol 9:1 (v/v) to afford 150. mg of the title compound as a brown oil (71% yield).
R*_{f}* = 0.28 (dichloromethane/methanol 9:1 (v/v)).
NMR Spectroscopy: ¹H NMR (500 MHz, CDCl₃, 23 °C, δ): 6.86 (dd, *J* = 9.1, 9.1 Hz, 1H), 6.69 (dd, *J* = 14.0, 2.8 Hz, 1 H), 6.59 (m, 1 H), 3.81 (s, 4H), 3.63 (br, 1 H), 3.05 (s, 4H). ¹³C NMR (125 MHz, CDCl₃, 23 °C, δ): 152.8 (d, *J* = 243.7 Hz), 146.3 (d, *J* = 7.8 Hz), 141.3 (d, *J* = 10.6 Hz), 114.6 (d, *J* = 2.9 Hz), 111.6 (d, *J* = 3.5 Hz), 105.7 (d, *J* = 21.1 Hz), 56.9 (s), 50.5 (s), 45.5 (s).
Mass Spectrometry: HRMS-FIA(m/z) calcd for C₁₁H₁₆FN₂O [M+H]⁺, 211.1247; found, 211.1245.

### 1-(4-(Benzyloxy)phenyl)piperazine (3k)

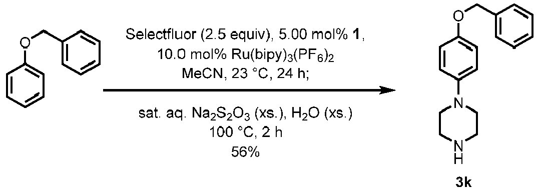

A 100 mL pressure tube was charged with palladium complex 1 (31.8 mg, 50.0 µmol. 5.00 mol%), Ru(bipy)₃(PF₆)₂ (86.0 mg, 100. µmol, 10.0 mol%), Selectfluor (886. mg, 2.50 mmol, 2.50 equiv), and benzyl phenyl ether (184. mg, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, 0.5 mL ethylene diamine was added and the reaction mixture was basified to pH 14 with 6 M NaOH, then filtered through celite. The filter cake was extracted with acetonitrile (5 × 15 mL). The acetonitrile was removed from the filtrate by rotary evaporation. The remaining aqueous mixture was transferred to a separatory funnel and extracted with dichloromethane (5 × 15 mL). The combined organic layers were extracted with 1 M HCl (5 × 15 mL). To the combined acidic aqueous layers was added ethylene diamine (0.5 mL), and the mixture was basified to pH 14 with 6 M NaOH. The basic aqueous layer was extracted with dichloromethane (5 × 15 mL). The combined organic layers were dried over sodium sulfate, filtered, and concentrated *in vacuo* to afford a red oil. The residue was purified by chromatography on silica gel eluting with dichloromethane/methanol 19:1 → 9:1 (v/v) to afford 151. mg of the title compound as an off-white powder (56% yield).
R*_{f}* = 0.35 (dichloromethane/methanol 9:1 (v/v)).
NMR Spectroscopy: ¹H NMR (600 MHz, CDCl₃, 23 °C, δ): 7.44-7.41 (m, 2H), 7.40-7.33 (m, 2H), 7.34-7.30 (m, 1H), 6.94-6.88 (m, 4H), 5.02 (s, 2H), 3.07 (s, 8H). ¹³C NMR (125 MHz, CDCl₃, 23 °C, δ): 153.1, 146.2, 137.3, 128.5, 127.8, 127.5, 118.3, 115.6, 70.5, 51.4, 46.0.
Mass Spectrometry: HRMS-FIA(m/z) calcd for C₁₇H₂₁N₂O [M+H]⁺, 269.1648; found, 269.1635.

### 2-Methoxy-5-(piperazin-1-yl)benzonitrile (3l)

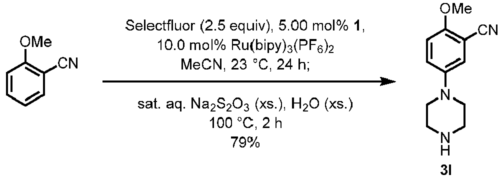

A 100 mL pressure tube was charged with palladium complex 1 (31.8 mg, 50.0 µmol.5.00 mol%), Ru(bipy)₃(PF₆)₂ (86.0 mg, 100. µmol, 10.0 mol%), Selectfluor (886. mg, 2.50 mmol, 2.50 equiv), and 2-methoxybenzonitrile (122. µL, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, 0.5 mL ethylene diamine was added and the reaction mixture was basified to pH 14 with 6 M NaOH, then filtered through celite. The filter cake was extracted with acetonitrile (5 × 15 mL). The acetonitrile was removed from the filtrate by rotary evaporation. The remaining aqueous mixture was transferred to a separatory funnel and extracted with dichloromethane (5 × 15 mL), then 9:1 dichloromethane/methanol (v/v) (3 × 15 mL). The combined organic layers were extracted with 1 M HCl (5 × 15 mL). To the combined acidic aqueous layers was added ethylene diamine (0.5 mL), and the mixture was basified to pH 14 with 6 M NaOH. The basic aqueous layer was extracted with 9:1 dichloromethane/methanol (v/v) (6 × 15 mL). The combined organic layers were dried over sodium sulfate, filtered, and concentrated *in vacuo* to afford a red oil. The residue was purified by chromatography on silica gel eluting with dichloromethane/methanol 9:1 (v/v) to afford 190. mg of the title compound as a brown oil (79% yield).
R*_{f}* = 0.31 (dichloromethane/methanol 9:1 (v/v)).
NMR Spectroscopy: ¹H NMR (500 MHz, CDCl₃, 23 °C, δ): 7.12 (dd, *J =* 9.1, 3.1 Hz, 1H), 7.07 (d, *J =* 3.1 Hz, 1 H), 6.88 (d, *J =* 9.1 Hz, 1 H), 3.86 (s, 4H), 3.04 (s, 4H), 2.56 (br, 1 H). ¹³C NMR (125 MHz, CDCl₃, 23 °C, δ): 155.2, 145.8, 123.2, 121.1, 116.7, 112.3, 101.7, 56.2, 50.9, 40.8.
Mass Spectrometry: HRMS-FIA(m/z) calcd for C₁₂H₁₆N₃O [M+H]⁺, 218.1293; found, 218.1300.

### Methyl 4-methoxy-3-(piperazin-1-yl)benzoate (3m)

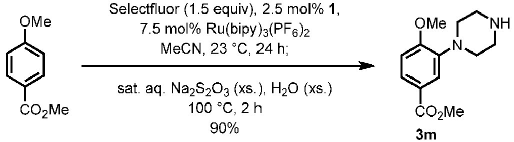

A 100 mL pressure tube was charged with palladium complex 1 (15.9 mg, 25.0 µmol. 2.50 mol%), Ru(bipy)₃(PF₆)₂ (64.5 mg, 75.0 µmol, 7.50 mol%), Selectfluor (531. mg, 1.50 mmol, 1.50 equiv), and methyl 4-methoxybenzoate (166. mg, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, ethylenediamine (1.5 mL) and water (15 mL) were added, and the mixture was stirred for 1 h further. The reaction mixture was transferred to a separatory funnel. Dichloromethane (20 mL) was added and the organic layer was separated. The aqueous layer was extracted with dichloromethane (2 × 15 mL). The combined organic layers were extracted with 10% aqueous glacial acetic acid (2 × 15 mL). The combined acidic aqueous layers were basified with ethylenediamine (4.5 mL). The basic aqueous layer was extracted with dichloromethane (3 × 15 mL). The combined organic layers were dried over sodium sulfate, filtered, and concentrated *in vacuo* to afford a red oil (252. mg). The residue was purified by chromatography on silica gel eluting with a solvent mixture of dichloromethane/methanol/28% aqueous ammonium hydroxide (91.5/8.0/0.5 (v/v/v)) to afford 226. mg of the title compound as a yellow solid (90% yield). R*_{f}* = 0.16 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 7.74 (dd, *J=* 8.1, 2.1 Hz, 1H), 7.61 (d, *J* = 2.4 Hz, 1 H), 6.88 (d, *J* = 8.4 Hz, 1 H), 3.92 (s, 3H), 3.88 (s, 3H), 3.09-3.11 (m, 8H), 1.67 (s, 1H). ¹³C NMR (125 MHz, CDCl₃, 23 °C, δ): 167.1, 156.2, 141.3, 125.6, 122.9, 119.7, 110.5, 55.8, 52.0, 51.6, 46.0.
Mass Spectrometry: HRMS-FIA (m/z) calcd for C₁₃H₁₈N₂O₃Na [M+Na]⁺, 273.1210; found, 273.1215.

### 2-(Piperazin-1-yl)-9H-fluoren-9-one (3n)

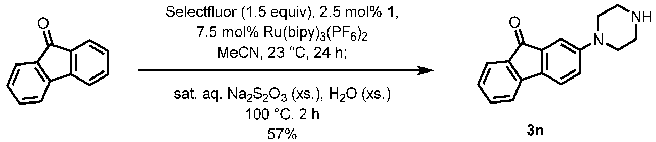

A 100 mL pressure tube was charged with palladium complex **1** (8.83 mg, 13.9 µmol. 2.50 mol%), Ru(bipy)₃(PF₆)₂ (35.8 mg, 41.6 µmol, 7.50 mol%), Selectfluor (295. mg, 0.832 mmol, 1.50 equiv), and fluorenone (100. mg, 0.555 mmol, 1.00 equiv). Acetonitrile (2.8 mL, c = 0.20 M) was added via syringe. The reaction mixture was stirred at 23 °C for 24 h. Saturated aqueous sodium thiosulfate (5.5 mL) and water (5.5 mL) were added, the pressure tube was sealed, and the reaction mixture was stirred at 100 °C for 2 h. After cooling to 23 °C, the reaction mixture was transferred to a separatory funnel. Dichloromethane (20 mL) and ethylenediamine (0.3 mL) were added and the organic layer was separated. The aqueous layer was extracted with dichloromethane (3 × 15 mL). The combined organic layers were extracted with 10% aqueous glacial acetic acid (3 × 15 mL). Ethylenediamine (3.0 mL) was added to the combined acidic aqueous layers, followed by basification with saturated aqueous sodium carbonate (5 mL). The basic aqueous layer was extracted with dichloromethane (4 × 15 mL). The combined organic layers were dried over sodium sulfate, filtered, and concentrated *in vacuo* to afford a red solid (102. mg). The residue was purified by chromatography on silica gel eluting with a solvent mixture of dichloromethane/methanol/28% aqueous ammonium hydroxide (96.0/3.5/0.5 (v/v/v)) to afford 83.1 mg of the title compound as a yellow solid (57% yield).
R*_{f}* = 0.34 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 7.57 (d, *J* = 7.3 Hz, 1 H), 7.40 (td, *J* = 7.4, 1.1 Hz, 1 H), 7.34-7.36 (m, 2H), 7.23 (d, *J =* 2.4 Hz, 1H), 7.15 (td, *J* = 7.4, 1.1 Hz, 1 H), 6.93 (dd, *J* = 8.2, 2.4 Hz, 1 H), 3.19-3.21 (m, 4H), 3.02-3.04 (m, 4H), 1.74 (s, 1 H). ¹³C NMR (125 MHz, CDCl₃, 23 °C, δ): 194.6, 152.9, 145.4, 135.7, 135.1, 134.9, 134.4, 127.6, 124.3, 121.2, 120.5, 119.5, 111.9, 50.1, 46.1.
Mass Spectrometry: HRMS-FIA(m/z) calcd for C₁₇H₁₇N₂O [M+H]⁺, 265.1335; found, 265.1341.

### 8-(Piperazin-l-yl)quinoline (3o)

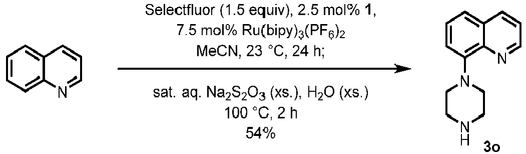

A 100 mL pressure tube was charged with palladium complex **1** (13.5 mg, 21.2 µmol. 2.50 mol%), Ru(bipy)₃(PF₆)₂ (54.6 mg, 63.5 µmol, 7.50 mol%), and Selectfluor (450. mg, 1.27 mmol, 1.50 equiv). Acetonitrile (4.2 mL, c = 0.20 M) was added, followed by quinoline (100. µL, 84.6 mmol, 1.00 equiv) via syringe. The reaction mixture was stirred at 23 °C for 24 h. Saturated aqueous sodium thiosulfate (8.5 mL) and water (8.5 mL) were added, the pressure tube was sealed, and the reaction mixture was stirred at 100 °C for 2 h. After cooling to 23 °C, the reaction mixture was transferred to a separatory funnel. Dichloromethane (20 mL) and ethylenediamine (2.5 mL) were added and the organic layer was washed with 6 M aqueous sodium hydroxide (5 mL). The aqueous layer was extracted with dichloromethane (2 × 15 mL). The combined organic layers were extracted with 1 M aqueous hydrochloric acid (2 × 15 mL). Ethylenediamine (5.0 mL) was added to the combined acidic aqueous layers, followed by basification with 6 M aqueous sodium hydroxide (5 mL). The basic aqueous layer was extracted with dichloromethane (3 × 15 mL). The combined organic layers were dried over sodium sulfate, filtered, and concentrated *in vacuo* to afford a red oil (137. mg). The residue was purified by chromatography on silica gel eluting with a solvent mixture of dichloromethane/methanol/28% aqueous ammonium hydroxide (91.5/8.0/0.5 (v/v/v)) to afford 98.2 mg of the title compound as a yellow oil (54% yield).

R*_{f}* = 0.18 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 8.86 (dd, *J =* 4.1, 1.8 Hz, 1 H), 8.08 (dd, *J =* 8.2, 1.8 Hz, 1H), 7.42-7.44 (m, 2H), 7.34 (dd, *J =* 8.2, 4.1 Hz, 1H), 7.12 (dd, *J* = 6.5, 2.4 Hz, 1 H). ¹³C NMR (125 MHz, CDCl₃, 23 °C, δ): 149.8, 148.3, 142.8, 136.6, 129.7, 126.8, 121.8, 120.9, 116.1, 53.2, 46.2.
Mass Spectrometry: HRMS-FIA(m/z) calcd for C₁₃H₁₆N₃ [M+H]⁺, 214.1339; found, 214.1328.

### 1-(2-Methoxypyridin-3-yl)piperazine (3p), 1-(6-methoxypyridin-3-yl)piperazine (3p-II)

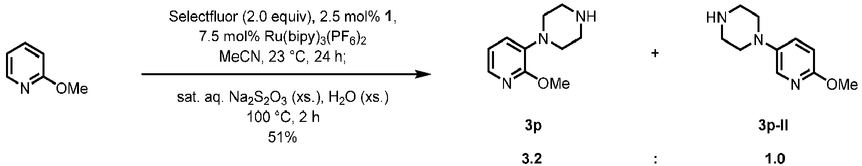

A 100 mL pressure tube was charged with palladium complex 1 (15.1 mg, 23.8 µmol. 2.50 mol%), Ru(bipy)₃(PF₆)₂ (61.3 mg, 71.3 µmol, 7.50 mol%), and Selectfluor (674. mg, 1.90 mmol, 2.00 equiv). Acetonitrile (4.8 mL, c = 0.20 M) was added, followed by 2-methoxypyridine (100. µL, 0.951 mmol, 1.00 equiv) via syringe. The reaction mixture was stirred at 23 °C for 24 h. Saturated aqueous sodium thiosulfate (9.5 mL) and water (9.5 mL) were added, the pressure tube was sealed, and the reaction mixture was stirred at 100 °C for 2 h. After cooling to 23 °C, the reaction mixture was transferred to a separatory funnel. Dichloromethane (15 mL) and ethylenediamine (2.5 mL) were added and the organic layer was washed with 6 M aqueous sodium hydroxide (5 mL). Volume of the aqueous layer was reduced by half and then the aqueous layer was extracted with dichloromethane (2 × 15 mL) and then mixture of dichloromethane/methanol (9.0/1.0 (v/v), 3 × 15 mL). The combined organic layers were extracted with 1 M aqueous hydrochloric acid (3 × 15 mL). Ethylenediamine (5.0 mL) was added to the combined acidic aqueous layers, followed by basification with 6 M aqueous sodium hydroxide (5 mL). The basic aqueous layer was extracted with dichloromethane (2 × 15 mL) and then mixture of dichloromethane/methanol (9.0/1.0 (v/v), 3 × 15 mL). The combined organic layers were dried over sodium sulfate, filtered, and concentrated *in vacuo* to afford a red oil (120. mg). The residue was purified by chromatography on silica gel eluting with a solvent mixture of dichloromethane/methanol/28% aqueous ammonium hydroxide (96.0/3.5/0.5 (v/v/v)) to afford 94.5 mg of the title compounds **(3p:3p-II)** as a yellow oil (51% yield).
R*_{f}* = 0.16 (dichloromethane/methanol/28% aqueous ammonium hydroxide 90.5:9.0:0.5 (v/v/v)).

NMR Spectroscopy: ¹H NMR (600 MHz, CDCl₃, 23 °C, δ): **3p** 7.76 (dd, *J* = 4.9, 1.7 Hz, 1 H), 7.05 (dd, *J* = 7.7, 1.7 Hz, 1 H), 6.80 (dd, *J =* 7.7, 4.9 Hz, 1 H), 3.95 (s, 3H), 2.98-3.02 (m, 8H), 2.19 (s, 1H). **3p-II** 7.74 (d, *J* = 3.0 Hz, 1 H), 7.24 (dd, *J* = 8.9, 3.0 Hz, 1 H), 6.64 (d, *J =* 8.9 Hz, 1 H), 3.84 (s, 3H), 2.98-3.02 (m, 8H), 2.19 (s, 1 H). ¹³C NMR (500 MHz, CDCl₃, 23 °C, δ): **3p** 156.9, 139.0, 136.6, 124.7, 117.1, 53.4, 51.3, 46.1. **3p-II** 158.9, 142.9, 134.6, 129.8, 110.7, 53.4, 51.6, 46.1.
Mass Spectrometry: HRMS-FIA(m/z) calcd for C₁₀H₁₆N₃O [M+H]⁺, 194.1293; found, 194.1295.

### 2-(4-(Piperazin-1-yl)phenyl)pyrimidine (3q)

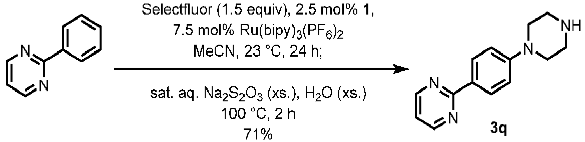

A 100 mL pressure tube was charged with palladium complex **1** (15.9 mg, 25.0 µmol. 2.50 mol%), Ru(bipy)₃(PF₆)₂ (64.5 mg, 75.0 µmol, 7.50 mol%), Selectfluor (531. mg, 1.50 mmol, 1.50 equiv), and 2-phenylpyrimidine (156. mg, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, the reaction mixture was basified with aqueous 6 M sodium hydroxide solution (10 mL) and ethylenediamine (0.5 mL), filtered through celite rinsing with acetonitrile (5 × 15 mL), and acetonitrile removed *in vacuo* to afford a brown aqueous solution. The aqueous layer was transferred to a separatory funnel and extracted with a solvent mixture of methanol/dichloromethane (1/9 (v/v), 5 × 15 mL). The combined organic layers were extracted with aqueous 1M hydrochloric acid solution (3 × 15 mL). The combined acidic aqueous layers were basified to pH 14 with aqueous 6M sodium hydroxide solution and ethylenediamine (0.5 mL). The basic aqueous layer was extracted with a solvent mixture of methanol/dichloromethane (1/9 (v/v), 5 × 15 mL). The organic layers were dried over sodium sulfate, filtered, and concentrated *in vacuo* to afford a red oil. The residue was purified by chromatography on silica gel eluting with a solvent mixture of dichloromethane/methanol (9/1 (v/v)) to to afford 171. mg of the title compound as an off-white solid (71% yield).
R*_{f}* = 0.08 (dichloromethane/methanol/28% aqueous ammonium hydroxide 90.5:9.0:0.5 (v/v/v)).

NMR Spectroscopy: ¹H NMR (500 MHz, DMSO-d₆, 23 °C, δ): 8.77 (d, *J* = 5.0 Hz, 2H), 8.23 (d, *J* = 9.5 Hz, 2H), 7.27 (t, *J* = 4.5 Hz, 1 H), 7.00 (d, *J* = 8.5 Hz, 2H), 3.21 (t, *J* = 5.0 Hz, 4H), 2.86 (t, *J =* 5.0 Hz, 4H). ¹³C NMR (125 MHz, DMSO-d₆, 23 °C, δ): 163.5, 157.4, 153.0, 128.8, 126.7, 118.4, 114.1, 47.9, 45.2.
Mass Spectrometry: HRMS-FIA(m/z) calcd for C₁₄H₁₇N₄ [M+H]⁺, 241.1448; found, 241.1457.

### 1-(4'-(Trimethylsilyl)-[1,1'-biphenyl]-4-yl)piperazine (3r)

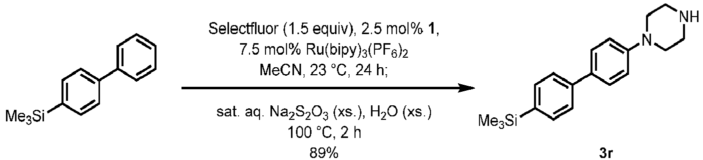

A 100 mL pressure tube was charged with palladium complex **1** (7.03 mg, 11.0 µmol. 2.50 mol%), Ru(bipy)₃(PF₆)₂ (28.5 mg, 33.1 µmol, 7.50 mol%), Selectfluor (235. mg, 0.662 mmol, 1.50 equiv), and [1,1'-biphenyl]-4-yltrimethylsilane (100. mg, 0.442 mmol, 1.00 equiv). Acetonitrile (2.2 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, ethylenediamine (2.5 mL) and water (20 mL) were added, and the mixture was stirred for 1 h further. The reaction mixture was transferred to a separatory funnel. Dichloromethane (20 mL) was added and the organic layer was separated. The aqueous layer was extracted with dichloromethane (3 × 15 mL). The combined organic layers were dried over sodium sulfate, filtered, and concentrated *in vacuo* to afford a red oil (220. mg). The residue was purified by chromatography on silica gel eluting with a solvent mixture of dichloromethane/methanol/28% aqueous ammonium hydroxide (97.5/2.0/0.5 (v/v/v)) to afford 122. mg of the title compound as a yellow solid (89% yield).
R*_{f}* = 0.31 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 7.56-7.60 (m, 4H), 7.53-7.55 (m, 2H), 6.99-7.02 (m, 2H), 3.21-3.23 (m, 4H), 3.06-3.08 (m, 4H), 2.16 (s, 1 H), 0.31 (s, 9H). ¹³C NMR (125 MHz, CDCl₃, 23 °C, δ): 151.2, 141.4, 138.2, 133.9, 132.3, 127.8, 125.9, 116.2, 50.2, 46.2, -0.9.
Mass Spectrometry: HRMS-FIA(m/z) calcd for C₁₉H₂₇N₂Si [M+H]⁺, 311.1938; found, 311.1950.

### 1-([1,1'-Biphenyl]-4-yl)piperazine (3s)

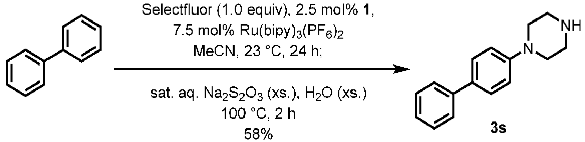

A 100 mL pressure tube was charged with palladium complex **1** (15.9 mg, 25.0 µmol. 2.50 mol%), Ru(bipy)₃(PF₆)₂ (64.5 mg, 75.0 µmol, 7.50 mol%), Selectfluor (531. mg, 1.00 mmol, 1.00 equiv), and 1,1'-biphenyl (154. mg, 1.00 mmol, 1.00 equiv). Acetonitrile (5.0 mL, c = 0.20 M) was added via syringe. The reaction mixture was stirred at 23 °C for 24 h. An aliquot was removed, concentrated, redissolved in CD₃CN, and analyzed by ¹H NMR, which showed a 9:1 mixture of biphenyl-TEDA:biphenyl, with no significant double TEDAylation product:

Saturated aqueous sodium thiosulfate (10 mL) and water (10 mL) were added, the pressure tube was sealed, and the reaction mixture was stirred at 100 °C for 2 h. After cooling to 23 °C, the reaction mixture was basified with aqueous 6 M sodium hydroxide solution (10 mL) and ethylenediamine (0.5 mL), filtered through celite rinsing with acetonitrile (5 × 15 mL), and acetonitrile removed *in vacuo* to afford a brown aqueous solution. The aqueous layer was transferred to a separatory funnel and extracted with a solvent mixture of methanol/dichloromethane (1/9 (v/v), 5 × 15 mL). The combined organic layers were extracted with aqueous 1M hydrochloric acid solution (3 × 15 mL). The combined acidic aqueous layers were basified to pH 14 with aqueous 6M sodium hydroxide solution and ethylenediamine (0.5 mL). The basic aqueous layer was extracted with a solvent mixture of methanol/dichloromethane (1/9 (v/v), 5 × 15 mL). The organic layers were dried over sodium sulfate, filtered, and concentrated *in vacuo* to afford a red oil. The residue was purified by chromatography on silica gel eluting with a solvent mixture of dichloromethane/methanol (9/1 (v/v)) to afford 138. mg of the title compound as an off-white solid (58% yield).
R*_{f}* = 0.10 (dichloromethane/methanol/28% aqueous ammonium hydroxide 90.5:9.0:0.5 (v/v/v)).
NMR Spectroscopy: ¹H NMR (600 MHz, F₃CO₂D, 23 °C, δ): 7.83 (m, 2H), 7.65 (m, 2H), 7.53 (m, 2H), 7.41 (m, 2H), 7.36 (m, 1H), 4.31 (s, 4H), 4.15 (s, 4H). ¹³C NMR (125 MHz, F₃CO₂D, 23 °C, δ): 149.6, 141.4, 133.0, 132.2, 132.0, 130.1, 123.6, 55.9, 45.8.
Mass Spectrometry: HRMS-FIA(m/z) calcd for C₁₆H₁₉N₂ [M+H]⁺, 239.1543; found, 239.1545.

### 1-(3-Fluoro-4-methoxyphenyl)piperazine (3t)

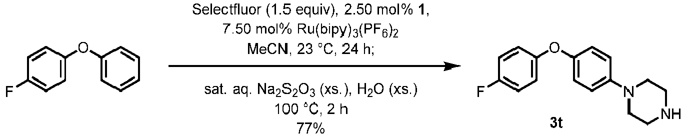

A 100 mL pressure tube was charged with palladium complex **1** (15.9 mg, 25.0 µmol. 2.50 mol%), Ru(bipy)₃(PF₆)₂ (64.5 mg, 75.0 µmol, 7.50 mol%), Selectfluor (532. mg, 1.50 mmol, 1.50 equiv), and phenyl 4-fluorophenyl ether (188. mg, 1.00 mmol, 1.00 equiv). Acetonitrile (5.0 mL, c = 0.20 M) was added via syringe. The reaction mixture was stirred at 23 °C for 24 h. Saturated aqueous sodium thiosulfate (10 mL) and water (10 mL) were added, the pressure tube was sealed, and the reaction mixture was stirred at 100 °C for 2 h. After cooling to 23 °C, the reaction mixture was filtered through celite, and the filter cake was extracted with 5 × 15 mL acetonitrile and 2 × 15 mL water. The acetonitrile was removed from the filtrate by rotary evaporation. To the remaining aqueous mixture 0.50 mL ethylene diamine was added, and the aqueous mixture was basified to pH 14 with 6 M NaOH, then transferred to a separatory funnel. The aqueous layer was extracted with dichloromethane (7 × 30 mL). The combined organic layers were extracted with 1 M HCl (5 × 30 mL). To the combined acidic aqueous layers was added ethylene diamine (0.5 mL), and the mixture was basified to pH 14 with 6 M NaOH. The basic aqueous layer was extracted with dichloromethane (5 × 30 mL). The combined organic layers were dried over sodium sulfate, filtered, and concentrated *in vacuo* to afford a red oil (220. mg). The residue was purified by chromatography on silica gel eluting with dichloromethane/methanol 19:1 → 9:1 (v/v) to afford 190. mg of the title compound as an off-white powder (77% yield).
R*_{f}* = 0.35 (dichloromethane/methanol 9:1 (v/v)).
NMR Spectroscopy: ¹H NMR (600 MHz, CDCl₃, 23 °C, δ): 7.00-6.95 (m, 2H), 6.94-6.88 (m, 6H), 3.74 (br, 1H), 3.19-3.12 (m, 4H), 3.12-3.06 (m, 4H). ¹³C NMR (125 MHz, CDCl₃, 23 °C, δ): 158.3 (d, *J =* 240.4 Hz), 154.1 (d, *J =* 2.5 Hz), 156.6 (s), 147.9 (s), 119.8 (s), 119.2 (d, *J =* 8.1 Hz), 117.9 (s), 116.0 (d, *J = 23.2* Hz), 50.6 (s), 45.7 (s).
Mass Spectrometry: HRMS-FIA(m/z) calcd for C₁₆H₁₈FN₂O [M+H]⁺, 273.1398; found, 273.1397.

### Methyl (S)-2-(1,3-dioxoisoindolin-2-yl)-3-(4-(piperazin-1-yi)phenyl)propanoate (3u)

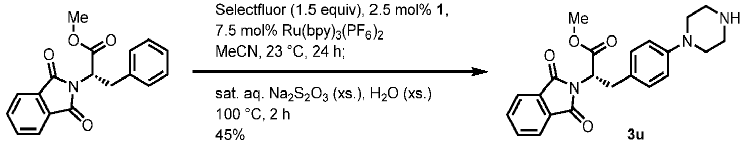

A 100 mL pressure tube was charged with palladium complex **1** (5.9 mg, 9.3 µmol. 2.5 mol%), Ru(bpy)₃(PF₆)₂ (24.0 mg, 27.9 µmol, 7.50 mol%), and Selectfluor (198 mg, 0.558 mmol, 1.50 equiv). Solution of methyl (*S*)-2-(1,3-dioxoisoindolin-2-yl)-3-phenylpropanoate (115 mg, 0.372 mmol, 1.00 equiv) in dry acetonitrile (1.9 mL, c = 0.20 M) was added via syringe. The reaction mixture was stirred at 23 °C for 24 h. Saturated aqueous sodium thiosulfate (3.8 mL) and water (3.8 mL) were added, the pressure tube was sealed, and the reaction mixture was stirred at 100 °C for 2 h. After cooling to 23 °C, water (5 mL) and saturated aqueous sodium carbonate (1 mL) were added, and the mixture was filtered over a glass frit with a filter paper. The filtrate was transferred to a separatory funnel. Dichloromethane (30 mL) was added and the organic layer was separated. The aqueous layer was extracted with dichloromethane (2 × 20 mL). The combined organic layers were dried over sodium sulfate, filtered, and concentrated *in vacuo* to afford a red solid (123 mg). The residue was purified by chromatography on silica gel eluting with a solvent mixture of dichloromethane/methanol/28% aqueous ammonium hydroxide (93.7/6.0/0.3 (v/v/v)) to afford 66.3 mg of the title compound as a yellow solid (45% yield).
R*_{f}* = 0.76 (dichloromethane/methanol/28% aqueous ammonium hydroxide 93.7:6.0:0.3 (v/v/v)). NMR Spectroscopy: ¹H NMR (600 MHz, CDCl₃, 23 °C, δ): 7.76-7.79 (m, 2H), 7.68-7.70 (m, 2H), 7.04-7.07 (m, 2H), 6.72-6.75 (m, 2H), 5.12 (dd, *J* = 11.0, 5.7 Hz, 1 H), 3.77 (s, 3H), 3.46-3.53 (m, 2H), 3.08-3.11 (m, 4H), 3.04-3.06 (m, 4H). ¹³C NMR (125 MHz, CDCl₃, 23 °C, δ): 169.6, 167.6, 150.1, 134.2, 131.8, 129.7, 128.4, 123.6, 116.7, 53.5, 53.0, 49.4, 45.5, 33.8. Mass Spectrometry: HRMS-FIA(m/z) calcd for C₂₂H₂₃N₃O₄ [M+H]+, 394.1761; found, 394.1760.

### Phenyl(5-(piperazin-1-yl)thiophen-2-yl)methanone (3v)

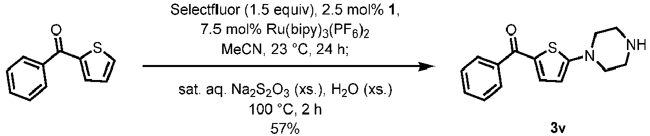

A 100 mL pressure tube was charged with palladium complex **1** (8.5 mg, 13. µmol. 2.5 mol%), Ru(bipy)₃(PF₆)₂ (34.2 mg, 40.0 µmol, 7.50 mol%), Selectfluor (282. mg, 0.800 mmol, 1.50 equiv), and phenyl(thiophen-2-yl)methanone (100. mg, 0.530 mmol, 1.00 equiv). Acetonitrile (2.6 mL, c = 0.20 M) was added via syringe. The reaction mixture was stirred at 23 °C for 24 h. Saturated aqueous sodium thiosulfate (5.3 mL) and water (5.3 mL) were added, the pressure tube was sealed, and the reaction mixture was stirred at 100 °C for 2 h. After cooling to 23 °C, the reaction mixture was transferred to a separatory funnel. Dichloromethane (20 mL), ethylenediamine (0.5 mL) and saturated aqueous sodium carbonate (5 mL) were added and the organic layer was separated. The aqueous layer was extracted with dichloromethane (3 × 15 mL). The combined organic layers were extracted with 1 M aqueous hydrochloric acid (3 × 10 mL). Ethylenediamine (6.0 mL) was added to the combined acidic aqueous layers, followed by basification with 3 M aqueous sodium hydroxide (6 mL). The basic aqueous layer was extracted with dichloromethane (4 × 15 mL). The combined organic layers were dried over sodium sulfate, filtered, and concentrated *in vacuo* to afford a red solid (126. mg). The residue was purified by chromatography on silica gel eluting with a solvent mixture of dichloromethane/methanol/28% aqueous ammonium hydroxide (95.5/4.0/0.5 (v/v/v)) to afford 82. mg of the title compound as a yellow solid (57% yield).
R*_{f}* = 0.38 (dichloromethane/methanol/28% aqueous ammonium hydroxide 90.5:9.0:0.5 (v/v/v)).
NMR Spectroscopy: ¹H NMR (600 MHz, CDCl₃, 23 °C, δ): 7.73 (d, *J*= 7.5 Hz, 2H), 7.49 (t, *J* = 7.5 Hz, 1 H), 7.42 (t, *J* = 7.5 Hz, 2H), 7.36 (d, *J* = 4.3 Hz, 1 H), 6.03 (d, *J* = 4.3 Hz, 1 H), 3.30 (t, J = 4.9 Hz, 4H), 2.99 (t, *J =* 4.9 Hz, 4H), 2.00 (s, 1 H). ¹³C NMR (125 MHz, CDCl₃, 23 °C, δ): 186.7, 167.8, 139.0, 138.3, 131.1, 128.7, 128.3, 127.5, 104.4, 50.6, 45.2. Mass Spectrometry: HRMS-FIA(m/z) calcd for C₁₅H₁₆N₂OS [M+H]⁺, 273.1056; found, 273.1046. Mass spectrometry: HRMS-FIA(m/z) calcd for C₁₅H₁₇N₂OS [M+H]⁺, 273.1056; found, 273.1044.

### Ethyl 5-(piperazin-1-yl)thiophene-2-carboxylate (3w)

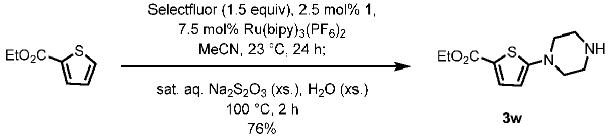

A 100 mL pressure tube was charged with palladium complex **1** (11.8 mg, 18.6 µmol. 2.50 mol%), Ru(bipy)₃(PF₆)₂ (47.9 mg, 55.7 µmol, 7.50 mol%), and Selectfluor (395. mg, 1.11 mmol, 1.50 equiv). Acetonitrile (3.7 mL, c = 0.20 M) was added, followed by ethyl thiophene-2-carboxylate (100. µL, 0.743 mmol, 1.00 equiv) via syringe. The reaction mixture was stirred at 23 °C for 24 h. Saturated aqueous sodium thiosulfate (7.4 mL) and water (7.4 mL) were added, the pressure tube was sealed, and the reaction mixture was stirred at 100 °C for 2 h. After cooling to 23 °C, the reaction mixture was transferred to a separatory funnel. Dichloromethane (20 mL) and ethylenediamine (0.5 mL) were added and the organic layer was separated. The aqueous layer was extracted with dichloromethane (2 × 15 mL). The combined organic layers were extracted with 10% aqueous glacial acetic acid (3 × 10 mL). The combined acidic aqueous layers were basified with ethylenediamine (4.5 mL). The basic aqueous layer was extracted with dichloromethane (3 × 15 mL). The combined organic layers were dried over sodium sulfate, filtered, and concentrated *in vacuo* to afford a red solid (156. mg). The residue was purified by chromatography on silica gel eluting with a solvent mixture of dichloromethane/methanol/28% aqueous ammonium hydroxide (95.5/4.0/0.5 (v/v/v)) to afford 135. mg of the title compound as a yellow solid (76% yield).
R*_{f}* = 0.52 (dichloromethane/methanol/28% aqueous ammonium hydroxide 90.5:9.0:0.5 (v/v/v)).
NMR Spectroscopy: ¹H NMR (500 MHz, CDCl₃, 23 °C, δ): 7.52 (d, *J* = 4.3 Hz, 1 H), 6.00 (d, *J =* 4.3 Hz, 1H), 4.25 (q, *J =* 7.1 Hz, 2H), 3.19-3.20 (m, 4H), 2.98-2.99 (m, 4H), 2.17 (s, 1 H), 1.30 (t, *J* = 7.1 Hz, 3H). ¹³C NMR (125 MHz, CDCl₃, 23 °C, δ): 165.2, 162.9, 134.9, 116.9, 104.1, 60.5, 50.8, 45.2, 14.5.
Mass Spectrometry: HRMS-FIA(m/z) calcd for C₁₁H₁₇N₂O₂S [M+H]⁺, 241.1005; found, 241.0995.

### Isopropyl-2-(4-(4-chlorobenzoyl)-2-(piperazin-1-yl)phenoxy)-2-methylpropanoate (3x)

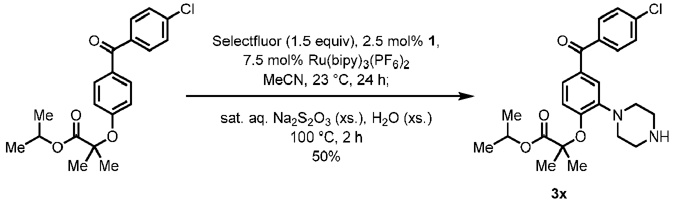

A 100 mL pressure tube was charged with palladium complex 1 (4.4 mg, 6.9 µmol. 2.5 mol%), Ru(bpy)₃(PF₆)₂ (17.9 mg, 20.8 µmol, 7.50 mol%), Selectfluor (147 mg, 0.416 mmol, 1.50 equiv), and fenofibrate (100 mg, 0.277 mmol, 1.00 equiv). Acetonitrile (1.4 mL, c = 0.20 M) was added via syringe, and the reaction mixture was stirred at 23 °C for 24 h. Saturated aqueous sodium thiosulfate (2.8 mL) and water (2.8 mL) were added, the pressure tube was sealed, and the reaction mixture was stirred at 100 °C for 2 h. After cooling to 23 °C, water (10 mL), saturated aqueous sodium carbonate (0.25 mL), and ethylenediamine (70 µL) were added, and the mixture was filtered over a glass frit with a filter paper. The filtrate was transferred to a separatory funnel. Dichloromethane (40 mL) was added and the organic layer was separated. The aqueous layer was extracted with dichloromethane (3 × 20 mL). The combined organic layers were dried over sodium sulfate, filtered, and concentrated *in vacuo* to afford a red solid (146 mg). The residue was purified by chromatography on silica gel eluting with a solvent mixture of dichloromethane/methanol/28% aqueous ammonium hydroxide (96.5/3.0/0.5 to 94.5/5.0/0.5 (v/v/v)) to afford 62 mg of the title compound as a yellow solid (50% yield). R*_{f}* = 0.11 (dichloromethane/methanol/28% aqueous ammonium hydroxide 95.5:4.0:0.5 (v/v/v)). NMR Spectroscopy: ¹H NMR (600 MHz, CD₃CN, 23 °C, δ): 7.69-7.71 (m, 2H), 7.51-7.53 (m, 2H), 7.37 (d, *J* = 2.2 Hz, 1 H), 7.31 (dd, *J* = 8.4, 2.2 Hz, 1 H), 6.73 (d, *J* = 8.4 Hz, 1H), 5.02 (hept, *J* = 6.2 Hz, 1H), 3.01-3.06 (m, 8H), 2.68 (bs, 1H), 1.65 (s, 6H), 1.18 (d, *J =* 6.2 Hz, 6H). ¹³C NMR (125 MHz, CD₃CN, 23 °C, δ): 195.0, 173.7, 153.4, 144.4, 138.6, 137.8, 132.2, 131.4, 129.4, 126.4, 121.1, 116.7, 80.8, 70.2, 51.7, 46.5, 25.6, 21.7. Mass Spectrometry: HRMS-FIA(m/z) calcd for C₂₄H₂₉ClN₂O₄ [M+H]+, 445.1889; found, 445.1888.

### TEDAylation of polymers

The TEDAylation reaction has been tested on two different polystyrenes with low molecular weights (800 and 2000) according to the above described procedure:

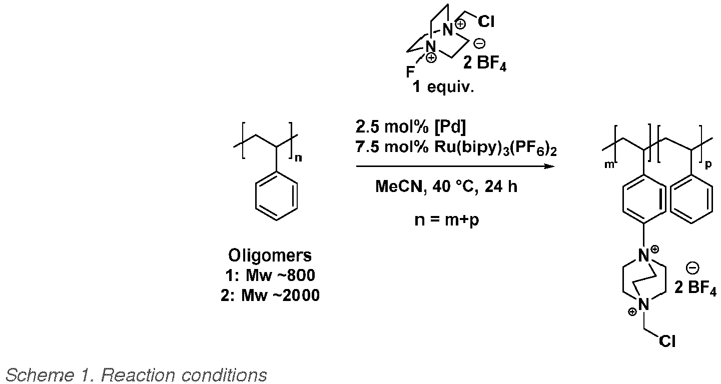

### Experimental procedure for both oligomers:

To a 5 mL vial were added palladium complex (2.99 mg, 4.7 µmol, 2.5 mol%), Ru(bipy)₃(PF₆)₂ (12.0 mg, 14 µmol, 7.5 mol%), Selectfluor (66.6 mg, 0.188 mmol, 1.00 equiv compared to styrene), and polystyrene (20 mg i.e. 0.188 mmol of styrene, 1.00 equiv), and acetonitrile (1 mL, c = 0.20 M). The reaction mixture was stirred at 40 °C for 24 h.

### Work-up of the reaction involving the PS Mw = 2000:

The reaction mixture was diluted with acetonitrile (2 mL), and dichloromethane was carefully added until a solid precipitated from the solution (1.5 mL). The resulting mixture was filtered on a glass frit, the solid was washed with dichloromethane (3 x 3 mL) and the filtrate was concentrated while the residue was dried under high vacuum (m = 42 mg). H-NMR of the crude and the two fractions showed the results discussed below. The H-NMR spectrum shows clearly a chemical modification on the aromatic rings, by comparison to the spectrum of the starting polystyrene. Moreover, two different signals corresponding to the TEDA moiety, one broad between 4 and 4.5 ppm and one sharp, which can be identified as the previously observed TEDA side product, confirm the functionalization of the polymer has been effective.

The precipitation of solids and subsequent washings allowed to eliminate the ruthenium ligand (NMR of the filtrate) and also some TEDA side-product, which is soluble in acetonitrile. The residue is now enriched in functionalized polymer, with still some side-product (NMR of the residue). Indeed, the ratio between the side-product and the TEDA grafted on the polymer is now around 0.27 (0.59 in the crude).

The residue was then dissolved in acetonitrile (1 mL) and dichloromethane (0.3 mL) was added, allowing to isolate 3 mg of solid after filtration/washings in dichloromethane. This new fraction is enriched in polymer, as observed by H-NMR (ratio = 0.23). The polymer is now soluble in acetonitrile and water (emulsion), and poorly soluble in chloroform, dichloromethane and toluene, which are known to be good solvents for polystyrenes. The number of TEDA moieties grafted on each chain has not yet been determined (mass spectroscopy is needed), but the solubility of the polymer is now similar to TEDA derivatives.

### Work-up of the reaction involving the PS Mw = 800:

Another work-up has been tested. After evaporation of the solvent and H-NMR of the crude, the crude was dissolved in acetonitrile (1 mL) and dichloromethane was added until a solid precipitated from the solution (0.6 mL). The vial was placed in a centrifuge (1 min at 5000 rpm) and the solvent was separated from the solids. To the vial containing the solids were added dichloromethane (0.6 mL) and acetonitrile (0.2 mL). The vial was placed in the centrifuge, the solvents were removed and the remaining solids were washed with dichloromethane.

A second purification using this time acetonitrile (0.7 mL) and toluene (0.4 mL, added until a precipitation was observed was performed. After centrifugation, the isolated solid was analyzed by HNMR, showing an efficient elimination of the TEDA side-product.

It has been observed that the functionalized polymer coming from the polystyrene Mw = 2000 is less soluble in a mixture of acetonitrile/dichloromethane than the other one, because of the rapid precipitation with the addition of dichloromethane. This observation shows that the more functionalized the polymer is, the more similar to the TEDA derivatives its properties are which is also related to the length of the polymer chains..

### Conclusion:

The TEDAylation reactions on oligomers (Mw of 800 and 2000) show promising results, with the observation by H-NMR of TEDA moieties grafted on the polymers (broad signals and changes in the aromatic protons area). The property of the isolated compound, still in mixture with side-products, is now different from the initial polystyrene. Indeed, the new polymer is more soluble in acetonitrile and water, while being insoluble in chloroform, dichloromethane and toluene.

As demonstrated above, an exceptional positional selectivity in aromatic substitution is achievable through a previously unappreciated phenomenon that radicals with high electron affinity undergo radical aromatic substitution with nearly exclusive para selectivity by eliciting significant arene-to-radical charge transfer in the transition state of addition. The inventive TEDAylation of aromatic compounds is a promising approach to a variety of new compounds and their use.

## Claims

1. An Ar-N-R¹-N'-diazoniabicyclo[2.2.2]octane salt of formula (I), 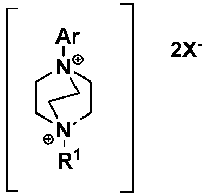 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or C₅ to C₂₀ heteroaryl-C₆ to C₂₀-alkyl group, each R¹ group being optionally substituted, and
X- is an anion.

2. The Ar-N-R¹-N'-diazoniabicyclo[2.2.2]octane salt of formula (I) as claimed in claim 1, wherein the mono- or polycyclic aromatic or heteroaromatic hydrocarbon Ar is substituted by one or more substituents R² which may be, independently from each other, the same or different and is each selected from hydrogen, halogen, straight chain, branched chain or cyclic aliphatic C₁ to C₂₀ alkyl group, optionally having one or more unsaturated bonds, C₆ to C₂₀ aryl or C₆ to C₂₀ aralkyl group or C₅ to C₂₀ heteroaryl or heteroaryl-C₆ to C₂₀-alkyl group, R² optionally bonded via -O- and/or R² optionally being substituted by one or more groups selected from halogen, straight chain, branched chain or cyclic aliphatic C₁ to C₂₀ alkyl group, optionally having one or more unsaturated bonds, C₆ to C₂₀ aromatic hydrocarbons or C₅ to C₂₀ heteroaromatic hydrocarbons, aryl-(C₁-C₆)-alkyl, or heteroaryl-(C₁-C₆)-alkyl, or at least two R² are forming a ring system selected from cyclic aliphatic C₄ to C₂₀ hydrocarbons, C₆ to C₂₀ aromatic hydrocarbons or C₅ to C₂₀ heteroaromatic hydrocarbons, each being optionally substituted, wherein at least one of R² is preferably not hydrogen.

3. The Ar-N-R¹-N'-diazoniabicyclo[2.2.2]octane salt of formula (I) as claimed in claim 1 or claim 2, wherein the mono- or polycyclic aromatic or heteroaromatic hydrocarbon Ar is bonded either directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof.

4. The Ar-N-R¹-N'-diazoniabicyclo[2.2.2]octane salt of formula (I) as claimed in claim 3, wherein the polymer is comprising more than one Ar- diazoniabicyclo[2.2.2]octane salt unit bonded either directly or via a linking group to the polymer.

5. Polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof, comprising at least two units Ar-N-R¹-N'-diazoniabicyclo[2.2.2]octane salt of formula (I), 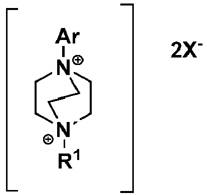 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or C₅ to C₂₀ heteroaryl-C₆ to C₂₀-alkyl group, each group being optionally substituted, and
X- is an anion.

6. The Ar-N-R¹-N'-diazoniabicyclo[2.2.2]octane salt of formula (I) as claimed in any of the preceding claims, wherein R¹ is a straight chain, branched chain or cyclic aliphatic C₁ to C₆ alkyl group, a C₆ to C₁₀ aryl or aralkyl group or C₅ to C₉ heteroaryl or heteroarylalkyl group, each group being optionally substituted.

7. The Ar-N-R¹-N'-diazoniabicyclo[2.2.2]octane salt of formula (I) as claimed in any of the preceding claims, wherein X- is an anion selected from halogen, triflate, BF₄⁻, SbF₆⁻, PtF₆⁻.

8. The Ar-N-R¹-N'-diazoniabicyclo[2.2.2]octane salt of formula (I) as claimed in any of the preceding claims, wherein R¹ does not have any hydrogen beta to the N'-nitrogen.

9. Process for preparing an Ar-N-R¹-N'-diazoniabicyclo[2.2.2]octane salt of formula (I) as claimed in any of claims 1 to 8,
wherein a compound H-Ar is reacted with 1-R¹-4-fluor-1,4-diazonia bicyclo [2.2.2] octane-bis-anion in the presence of a catalyst to yield a compound of the formula (I) 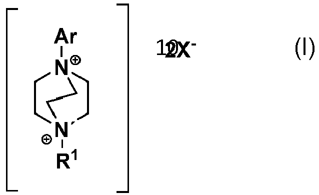 wherein
Ar is a mono- or polycyclic aromatic or heteroaromatic hydrocarbon, optionally being substituted by one or more substituents,
R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or heteroaryl-C₆ to C₂₀-alkyl group, each group being optionally substituted, and
X- is an anion.

10. Process for preparing a polymer comprising at least one Ar-N-R¹-N'-diazoniabicyclo-[2.2.2]octane salt unit of formula (I) as claimed in claim 5, wherein at least one Ar is bonded directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof,
wherein a polymer having said at least one mono- or polycyclic aromatic or heteroaromatic hydrocarbon Ar, optionally being substituted by one or more substituents, bonded to the polymer chain is reacted with an excess of 1-R¹-4-fluor-1,4-diazonia bicyclo [2.2.2] octane-bis-anion in the presence of a catalyst to yield a polymer comprising at least one Ar-N-R¹-N'-diazoniabicyclo-[2.2.2]octane salt unit of formula (I), wherein Ar, R¹ and X have the meanings as given above.

11. Process for preparing a polymer comprising at least two Ar-N-R¹-N'-diazoniabicyclo-[2.2.2]octane salt units of formula (I) as claimed in claim 5, wherein at least two Ar are bonded directly or via a linking group to a polymer, preferably selected from polyalkylenes such as polyethylene, polypropylene, polybutadiene, substituted polyalkylene, polyacrylate, polymethacrylate, polyester, polyethylene glycol, polypropylene glycol or copolymers thereof,
wherein at least two Ar-N-R¹-N'-diazoniabicyclo[2.2.2]octane salt units of formula (I), 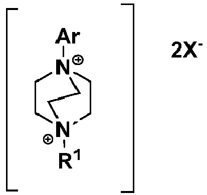 wherein
Ar is a mono- or polycyclic aromatic or heteroaromatic hydrocarbon, optionally being substituted by one or more substituents, Ar having a substituent R² with a polymerizable group, are reacted to polymerize via the polymerizable group, wherein Ar, R¹ and X have the meanings as given above.

12. Process according to claim 9 or 10, wherein the catalyst is a single electron redox catalyst used alone or in a mixture of two or more, preferably selected from Cu^{II}X and Ru^{II} X₂ catalysts, such as Ru(bipy)₃(PF₆)₂ , preferably in combination with a Pd catalyst of the formula:

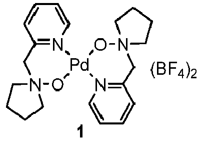

13. Process for preparing an Ar-piperazine as represented by the following formula (II): 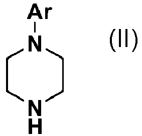 wherein a Ar-N-R¹-N'-diazoniabicyclo[2.2.2]octane salt of formula (I) as claimed in any of claims 1 to 8, 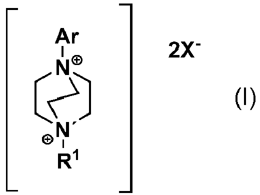 is reacted with a reducing agent, preferably sodium thiosulfate in an aqueous solution and preferably at an elevated temperature, to yield the Ar-piperazine as target compound, wherein Ar, R¹ and X have the meanings as given above.

14. An Ar-piperazine as represented by the following formula (II): 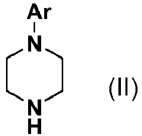 wherein Ar has the meaning as given above, obtainable according to the process of claim 12.

15. Use of the Ar-piperazine as represented by the following formula (II): 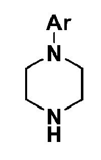
wherein Ar has the meaning as given above, or
of a Ar-N-R¹-N'-diazoniabicyclo[2.2.2]octane salt of formula (I) as claimed in any of claims 1 to 8, 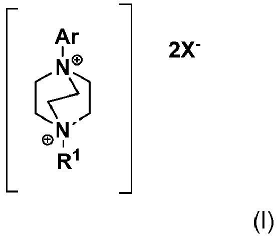
wherein Ar, R¹ and X have the meanings as given above,
for preparing functionalized polymers.

16. Use of a compound of the formula (III) 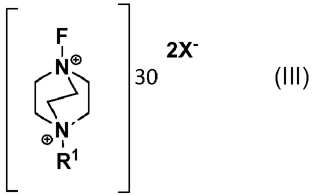 for a process for charge transfer directed radical substitution of aromatic or hetero aromatic compounds, wherein R¹ is a straight chain, branched chain or cyclic aliphatic C₁ to C₂₀ alkyl group, a C₆ to C₂₀ aryl or C₆ to C₂₀ aralkyl group or C₅ to C₂₀ heteroaryl or C₅ to C₂₀ heteroaryl-C₆ to C₂₀-alkyl group, each group being optionally substituted, and X- is an anion.

17. A functionalized polymer, obtainable by making use of the methods as claimed in claims 15 or 16.
